# EUROPEAN PATENT SPECIFICATION

(11) **EP 2 755 910 B1**
(45) Date of publication and mention of the grant of the patent: **20.05.2020**
(21) Application number: 12772507.5
(22) Date of filing: 12.09.2012
(51) Int. Cl.: C01B 13/02, A61M 16/10, A61M 16/00, A61M 16/06

(54) **PORTABLE OXYGEN CONCENTRATOR**
TRAGBARER SAUERSTOFFKONZENTRATOR
CONCENTRATEUR PORTABLE D'OXYGÈNE

(30) Priority: 13.09.2011 US 201161533874 P
(43) Date of publication of application: 23.07.2014
(73) Proprietor: Koninklijke Philips N.V., 5656 AG Eindhoven (NL)
(72) Inventor: WHITCHER, Douglas Adam, NL-5656 AE Eindhoven (NL); KOEPPEL, Bradley Stewart, NL-5656 AE Eindhoven (NL); HABERLAND, Bernhard Lewis, NL-5656 AE Eindhoven (NL); BLAIR, Jeremy, NL-5656 AE Eindhoven (NL)
(74) Representative: Philips Intellectual Property & Standards
(86) International application number: PCT/IB2012/054732
(87) International publication number: WO 2013/038342

(56) References cited:
- EP-A1- 1 637 209
- EP-A2- 1 598 103
- WO-A2-2007/118054
- US-A1- 2005 072 298

## Description

### FIELD OF THE INVENTION

The present disclosure pertains to a system and method for providing oxygen, and, in particular, a portable and efficient apparatus for concentrating oxygen by adsorption from air and methods for using such apparatus.

### BACKGROUND OF THE INVENTION

Patient suffering from lung diseases often need supplemental oxygen to improve their comfort and/or quality of life. Stationary sources of oxygen are available, e.g., oxygen lines in hospitals or other facilities that may provide oxygen to patients. To allow some mobility, cylinders of pure and/or concentrated oxygen can be provided that a patient may carry or otherwise take with them, e.g., on pull-along carts. Such cylinders, however, have limited volume and are large and heavy, limiting the patient's mobility.

Portable devices have been suggested that concentrate oxygen from ambient air to provide supplemental oxygen. For example, U.S. Patent Nos. 5,531,807 6,520,176, 6,764,534, 7,368,005, 7,402,193, 7,794522, and 7,837,761 disclose portable oxygen concentrators that separate nitrogen from ambient air, and deliver a stream of concentrated oxygen that may be stored in a tank or delivered directly to patients.

It is further known from EP 1 598 103 a system for producing an oxygen-rich gas comprising (a) a primary gas mover including a first and a second compressor, wherein the primary gas mover is characterized by a weight Wp; (b) a drive motor adapted to drive the first and second compressors; (c) a rechargeable power supply characterized by a weight, Wb; and (d) a pressure/vacuum swing adsorption unit adapted to separate the pressurized feed air into an oxygen-rich product at a product flow rate Fp and an oxygen-depleted waste gas, wherein the adsorption unit comprises a plurality of adsorber beds containing an adsorbent and characterized by a total adsorbent weight Wa; and wherein the combined weight, Wt, of the adsorbent, the primary gas mover, and the rechargeable power supply is characterized by the expression where Fp is in liters per min (at 23°C and 1 atma pressure), and Wa, Wp, and Wb are in pounds.

### SUMMARY OF THE INVENTION

It is an object of one or more embodiments to provide an oxygen concentrator system as from claim 1.

It is yet another aspect of one or more embodiments to provide a method of 15 concentrating oxygen using the system of claim 1. The method includes generating a supply of compressed air from ambient air; generating a supply of oxygen-enriched gas from the supply of compressed air; and communicating oxygen-enriched gas from the generated supply of oxygen-enriched gas to a respiratory circuit for delivery to an airway of a subject, wherein a ratio R_{OW} is determined as: R_{OW} = (O₂ output)/total weight of the oxygen concentrator system, where O₂ output is the maximum continuous oxygen communicated to the respiratory circuit, and wherein the ratio R_{OW} is greater than about 0.18 lpm/lbs.

These and other objects, features, and characteristics of the present embodiments, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of any limits.

### BRIEF DESCRIPTION OF THE DRAWINGS

FIG. 1 is a side cross-sectional view of a oxygen concentrator system (for sake of clarity compressor, air inlet filter, sieve beds, reservoir and controller are not shown) in accordance with an embodiment of the present disclosure;
FIG. 2 schematically illustrates the oxygen concentrator system in accordance with an embodiment of the present disclosure;
FIG. 3 is a rear view of a support member (or a central chassis) of the oxygen concentrator system with integrally formed upper (oxygen) and lower (air) manifolds in accordance with an embodiment of the present disclosure;
FIG. 4A is a perspective view of a first side surface of the support member in accordance with an embodiment of the present disclosure;
FIG. 4B is a perspective view of a second side surface of the support member with integrally formed manifolds and their respective cover members in accordance with an embodiment of the present disclosure;
FIG. 5 is another perspective view of the second side surface of the support member with air control valves being attached thereon in accordance with an embodiment of the present disclosure;
FIG. 6 is a partial perspective view of the second side surface of the support member with check valves being attached thereon in accordance with an embodiment of the present disclosure;
FIG. 7 shows a perspective view of the first side surface of the support member with oxygen side balance valve being attached thereon in accordance with an embodiment of the present disclosure;
FIG. 8 shows another perspective view of the first side surface of the support member with air inlet filter being attached thereon in accordance with an embodiment of the present disclosure;
FIG. 9 shows another perspective view of the first side surface of the support member with compressor being attached thereon in accordance with an embodiment of the present disclosure;
FIGS. 10 shows another perspective view of the first side surface of the support member with tubing to the compressor and tubing from the compressor to air manifold being attached to the support member in accordance with an embodiment of the present disclosure;
FIG. 11 shows another perspective view of the first side surface of the support member with outlet air filter and muffler being attached thereon in accordance with an embodiment of the present disclosure;
FIG. 12 shows another perspective view of the first side surface of the support member with noise shield being attached thereon in accordance with an embodiment of the present disclosure;
FIG. 13 shows another perspective view of the first side surface of the support member with sieve beds being attached thereon in accordance with an embodiment of the present disclosure;
FIG. 14 is a perspective view of a housing member of the oxygen concentrator system and the support member with reservoir, valves, and controller disposed thereon in accordance with an embodiment of the present disclosure; and
FIG. 15 is another perspective view of the housing member and the support member of the oxygen concentrator system in accordance with an embodiment of the present disclosure.
FIG. 16 illustrates a method of concentrating oxygen in accordance with an embodiment of the present disclosure.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein. In this specification the following non-SI units are used, which may be converted to the respective SI or metric unit according to the following conversion table:
Name of unit Symbol Conversion factor SI unit
Pounds lb 453.6 gram inch in 2.4 mm

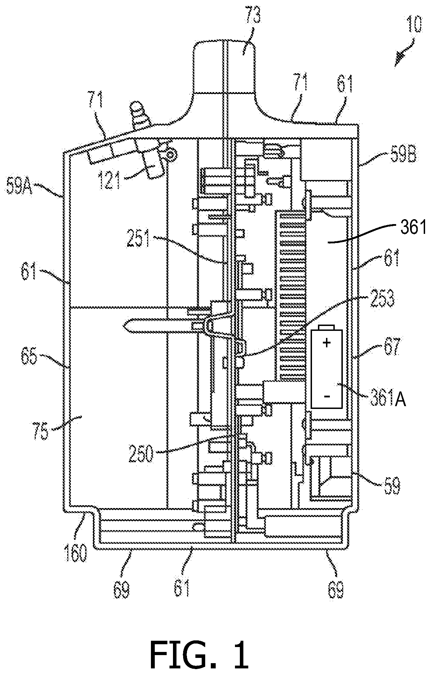

FIGS. 1-4B show an exemplary embodiment of an oxygen concentrator system 10. Oxygen concentrator system 10 may include an oxygen concentration subsystem 13 configured to implement adsorption to generate a supply of oxygen-enriched gas, an oxygen delivery subsystem 11 configured to communicate oxygen-enriched gas from the oxygen concentration subsystem to a respiratory circuit for delivery to an airway of a subject, one or more batteries 361A, provided in battery compartment 361, wherein the one or more batteries 361 are configured to act as a sole power supply for oxygen concentrator system 10, and a housing configured to house oxygen concentration subsystem 13, oxygen delivery subsystem 11, and one or more batteries 361A. The illustration of battery 361A as a single component in FIG. 1 is not intended as a limiting example.

Oxygen concentration subsystem 13 includes one or more of a compressor 14, one or more sieve beds 12 configured to adsorp nitrogen from air, comprising a first port 32 and a second port 34, such that the sieve bed produces oxygen-enriched gas, and/or other components. Compressor 14 is configured to deliver pressurized air to first port 32 of one or more sieve beds 12. Oxygen delivery subsystem 11 may include one or more of a reservoir 18 configured to store oxygen-enriched gas exiting from one or more sieve beds 12, a support member 250, which may form a central chassis or spine, positioned in housing 59 and configured to support compressor 14, sieve beds 12 and, if present, reservoir 18. The oxygen delivery subsystem 11 includes an air manifold 16 providing a plurality of channels 67 therein that at least partially define inlet air passages 64-68 communicating between compressor 14 and first ports 32 of the one or more sieve beds 12, and an oxygen delivery manifold 102 providing a plurality of channels 67 therein that at least partially define passages 108 for delivering the oxygen-enriched gas to a user. Air manifold 16 and oxygen delivery manifold 102 may be integrally formed with support member 250. The user of the oxygen concentrator system may interchangeably be referred to herein as the subject.

Optionally, oxygen concentrator system 10 may include one or more additional components, e.g., one or more check valves, filters, sensors, additional electrical power sources (not shown), and/or other components, at least some of which may be coupled to a controller 22 (and/or one or more additional controllers, also not shown), as described further below. It will be appreciated that the terms "airflow," "air," or "gas" may be used generically herein, even though the particular fluid involved may be ambient air, pressurized nitrogen, concentrated oxygen, and the like.

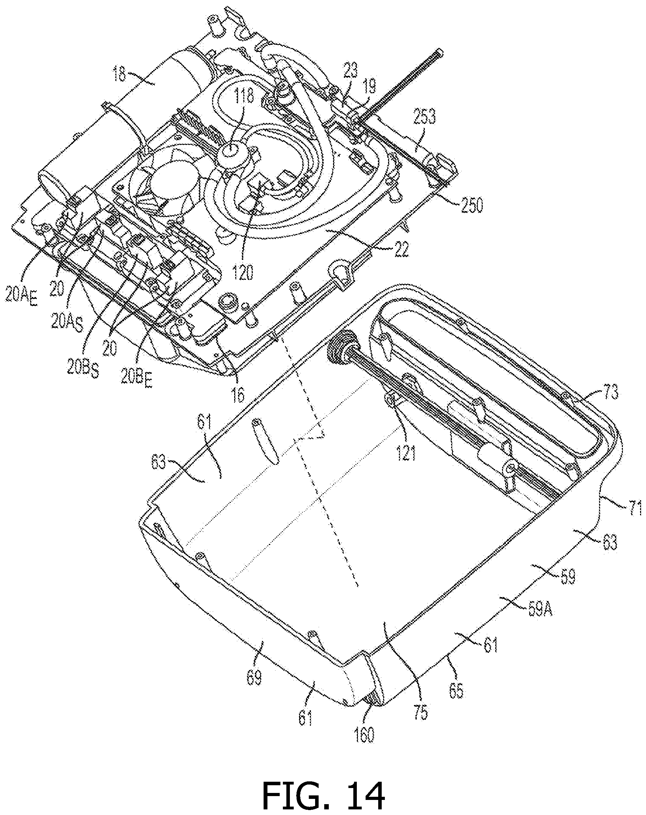

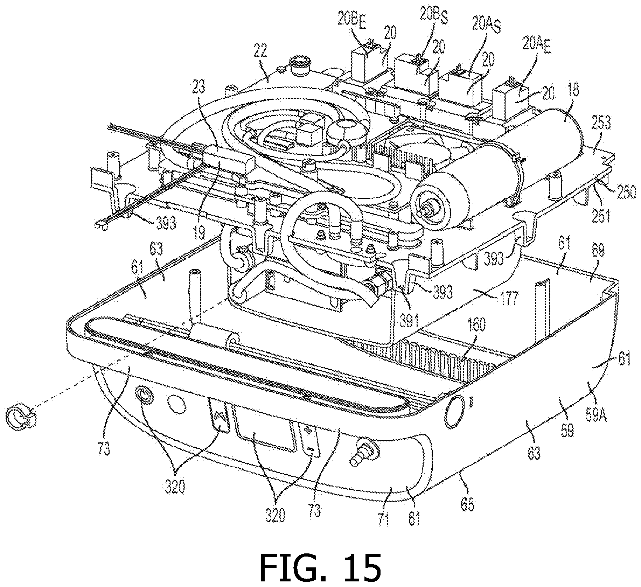

As shown in FIG. 1, housing 59 of oxygen concentrator system 10 includes a plurality of walls 61 that may define outer structural surface of oxygen concentrator system 10. Plurality of walls 61 may include a pair of side walls 63 (FIGS. 14 and 15), a front wall 65, a top wall 71, a bottom wall 69, and a rear wall 67. Plurality of walls 61 may define a volume of oxygen concentrator 10. Oxygen concentrator system 10 may include a carrying handle 73 connected to at least one of walls 61 (e.g., top wall 71) to enable oxygen concentrator system 10 to be transported.

In one embodiment, housing 59 may be formed of at least two mating housing members 59A and 59B cooperating with each other to define a hollow interior 75 therein. Hollow interior 75 of housing 59 may house support one or more of member 250, sieve beds 12, reservoir 18, compressor 14 and other components of oxygen concentrator system 10. First mating housing member 59A includes front wall 65, and at least a portion of side walls 63, bottom wall 69, top wall 71, and carrying handle 73, while second mating member 59B includes rear wall 67, and at least a portion of side walls 63, bottom wall 69, top wall 71, and carrying handle 73. First mating housing member 59A and the second mating housing member 59B may be connected to each other using any known attachment mechanism, for example, using fasteners. In another embodiment, support member 250 (with components of oxygen concentrator system 10 attached thereon) is first connected to mating housing member 59B and the assembly of mating housing member 59B and support member 250 is then connected to mating housing member 59A.

In one embodiment, side walls 63 and/or bottom wall 69 may include one or more inlet openings 160 (FIGS. 14 and 15) that may communicate with hollow interior 75 of oxygen concentrator system 10. Inlet openings 160 are configured to allow ambient air to pass easily through inlet openings 160, yet preventing large objects from passing therethrough.

Referring to FIGS. 1, 3-5 and 14-15, support member 250, which may form a central chassis or spine, may be configured to support one or more of compressor 14, sieve beds 12, reservoir 18 and/or other components of oxygen concentrator system 10. Support member 250 with compressor 14, sieve beds 12, reservoir 18 and/or other components of oxygen concentrator system 10 attached thereon may be disposed centrally in hollow interior 75 of housing 59.

Support member 250 may be formed from any engineering grade material, e.g., plastic, such as ABS, polycarbonate or composite materials. Support member 250 may be formed by injection molding, and the like. In another embodiment, support member 250 may be made from aluminum material or any other material suitable for machining or casting. It is noted that the material used to form support member 250, or any other component of oxygen concentrator 10, may substantially alter the total weight of oxygen concentrator system 10. Such a change in weight in turn changes any characteristic ratio of oxygen concentrator system 10 that includes the total weight.

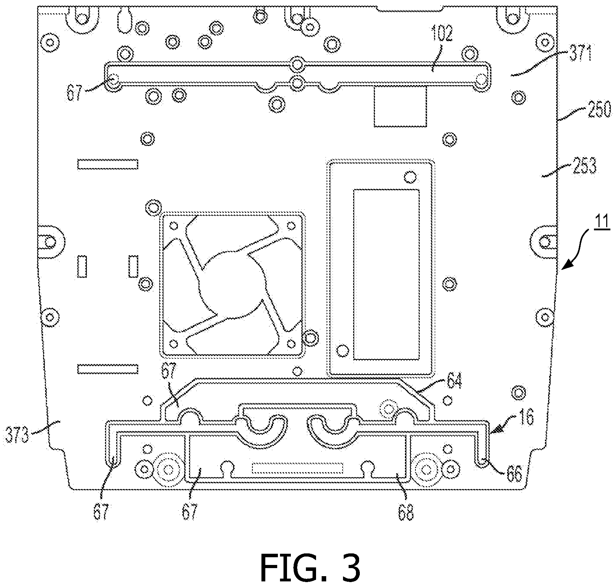

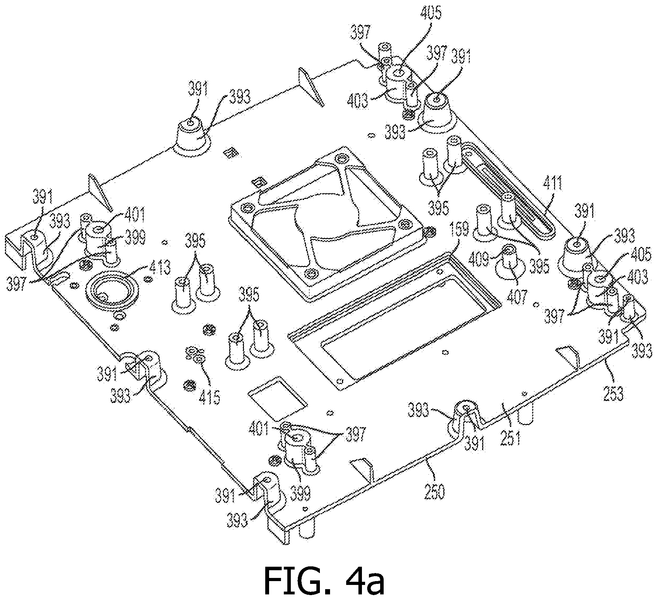

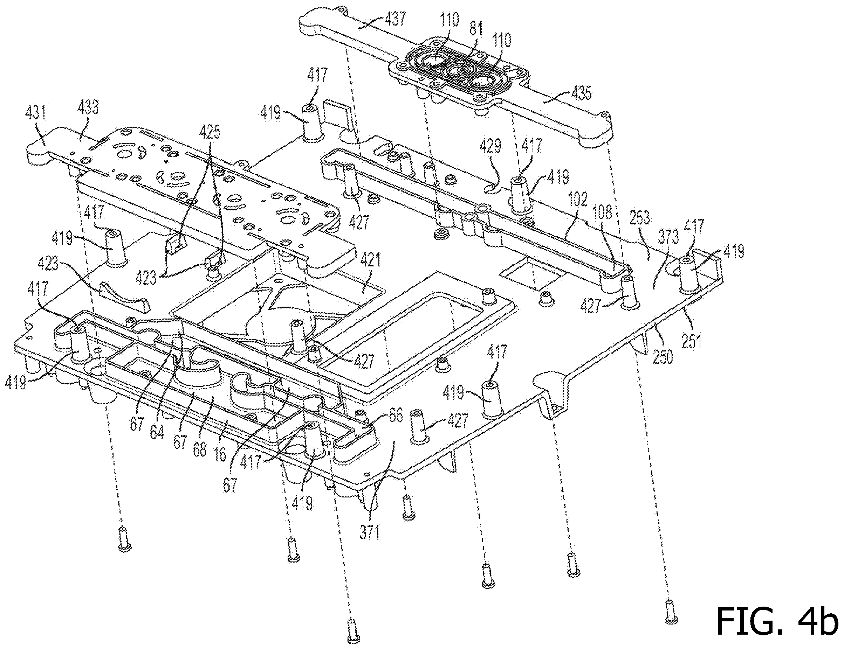

Referring to FIGS. 3-5 and 14-15, support member 250 with compressor 14, sieve beds 12, reservoir 18 and/or other components (e.g., controller 22, valves, etc.) of oxygen concentrator system 10 attached thereon may first be attached to, for example, mating housing member 59A using fasteners. To attach support member 250 to mating housing member 59A, fasteners maybe installed through holes 391 in attachment members 393. Then, mating housing member 59A along with support member 250 (and components of oxygen concentrator system 10 attached thereon) may be attached to mating housing member 59B using fasteners. To attach mating housing member 59A to mating housing member 59A, fasteners may be installed through hole 417 in attachment members 419 of support member 250. Attachment members 393 with hole 391 are shown in FIG. 4A, while attachment member 419 with hole 417 are shown in FIG. 4B. In one embodiment, attachment members 393 and attachment member 419 are integrally formed or molded with support member 250.

Support member 250 of oxygen concentrator system 10 has a first side surface 251 and a second side surface 253. First side surface 251 of support member 250 is shown in FIGS. 4A, and 7-13, while second side surface 253 of support member is shown in FIGS. 3 and 4B. Compressor 14 and sieve beds 12 maybe located on first side surface 251 of support member 250, while one or more of reservoir 18 and air control valves 20 may be located on second side surface 253 of support member 250.

As shown in FIGS. 3, and 4B, air manifold 16 may be integrally formed at a lower portion 371 of support member 250 and oxygen delivery manifold 102 may be integrally formed at an upper portion 371 of support member 250. Integrally forming air manifold 16 and oxygen delivery manifold 102 may obviate the need for at least some of the separate and discrete components typically used as pneumatic manifolds, plus associated tubing, valves, etc. Integrally forming pneumatic manifolds may reduce one or both of the total weight and/or the total volume of oxygen concentrator system 10. In one embodiment, air manifold 16 and oxygen delivery manifold 102 are integrally formed on second side surface 253 of support member 250.

As will be described below, air manifold 16 may include inlet air passages 64 and 66 for air to enter one or more sieve beds 12 and includes exit passage 68 for nitrogen to be exhausted out of the one or more sieve beds 12 into the atmosphere. Oxygen delivery manifold 102 may include passage 108 for oxygen-enriched gas from second ports 34 of the one or more sieve beds 12 to reservoir 18. Oxygen delivery manifold 102 also may include passage 108 and a passage 109 for oxygen-enriched gas from reservoir 18 to a respiratory circuit 111 for delivering the oxygen-enriched gas to a user. In some embodiments, oxygen concentrator system 10 may not include a reservoir, and thus communicate oxygen-enriched gas from the one or more sieve beds to respiratory circuit 11 for delivering the oxygen to a user. In some embodiments, respiratory circuit 111 may comprise a nasal cannula.

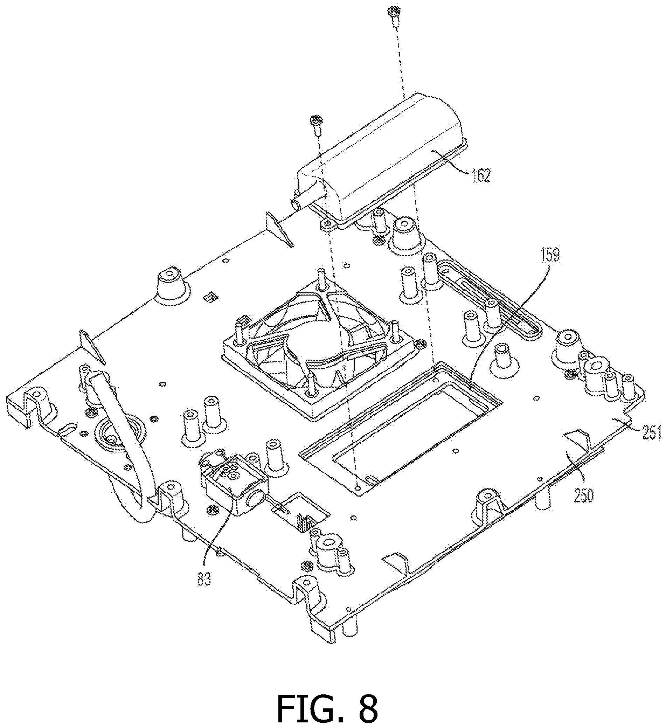

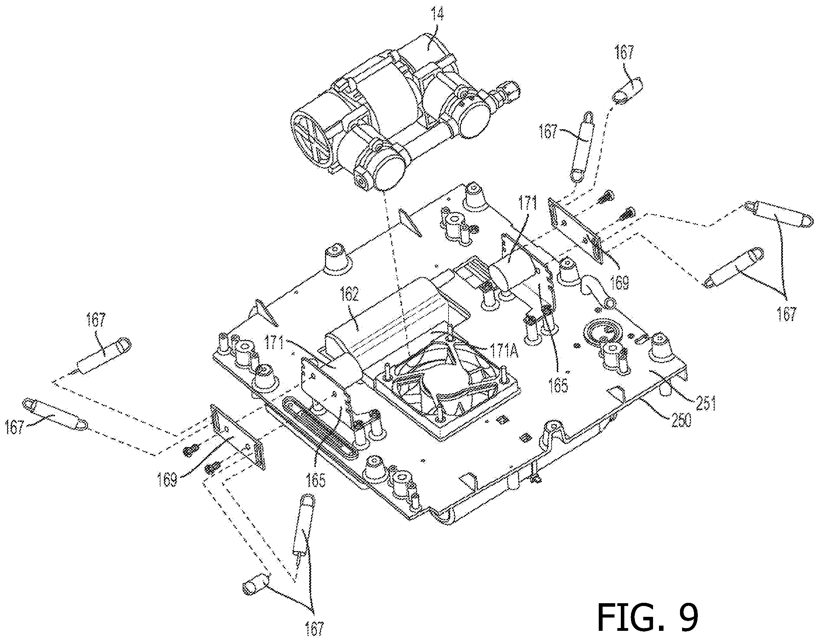

FIG. 4A shows a perspective view of first side surface 251 of support member 250. A recess 159 on first side surface 251 is configured to receive an inlet air filter 162 (shown in and described with respect to FIG. 8) therein. Bracket members 165 (shown in and described with respect to FIG. 9) may be used to position compressor 14 on support member 250. Bracket members 165 maybe attached to support member 250 using, for example, fasteners installed through holes in attachment members 395 of support member 250. In one embodiment, attachment members 395 are integrally formed or molded with support member 250.

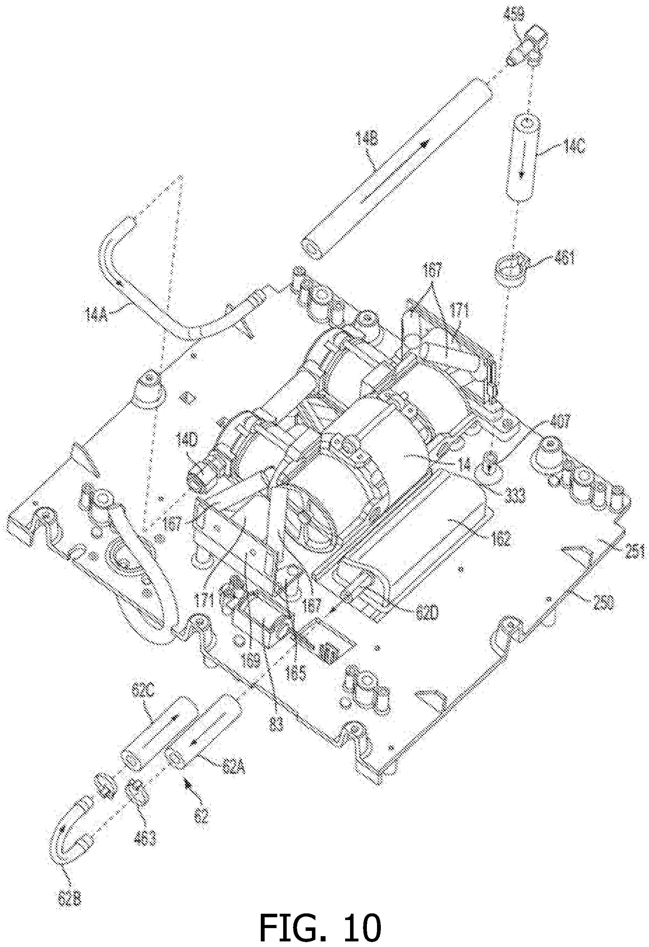

Compressed air from compressor 14 may enter a compressor outlet passage 64 of air manifold 16 through a first compressed air passage member 407. That is, first compressed air passage member 407 with an opening 409 therethrough is configured to direct or guide compressed air from a compressor outlet end 14D (and through passage members 14A-C as shown in and described with respect to FIG. 10) to compressor outlet passage 64 of air manifold 16. In some embodiments, first compressed air passage member 407 may be integrally formed or molded with support member 250.

One or more sieve beds 12 may be attached to side surface 251 of support member 250 using fasteners installed through holes in attachment members 397 of support member 250.

Compressed air from a sieve bed inlet passage 66 of air manifold 16 enters first ports 32 of the one or more sieve beds 12 through second compressed air passage members 403. That is, second compressed air passage members 403 with an openings 405 therethrough are configured to direct or guide compressed air from sieve bed inlet passage 66 of air manifold 16 to first port(s) 32 of the one or more sieve beds 12.

Oxygen from second port(s) 34 of the one or more sieve beds 12 enters oxygen delivery manifold 102 through oxygen passage members 399. That is, oxygen passage members 399 with openings 401 therethrough are configured to direct or guide oxygen from second port(s) 34 of the one or more sieve beds 12 into oxygen delivery manifold 102. In some embodiments, oxygen passage members 399 and/or a set of second compressed air passage members 403 may be integrally formed or molded with support member 250.

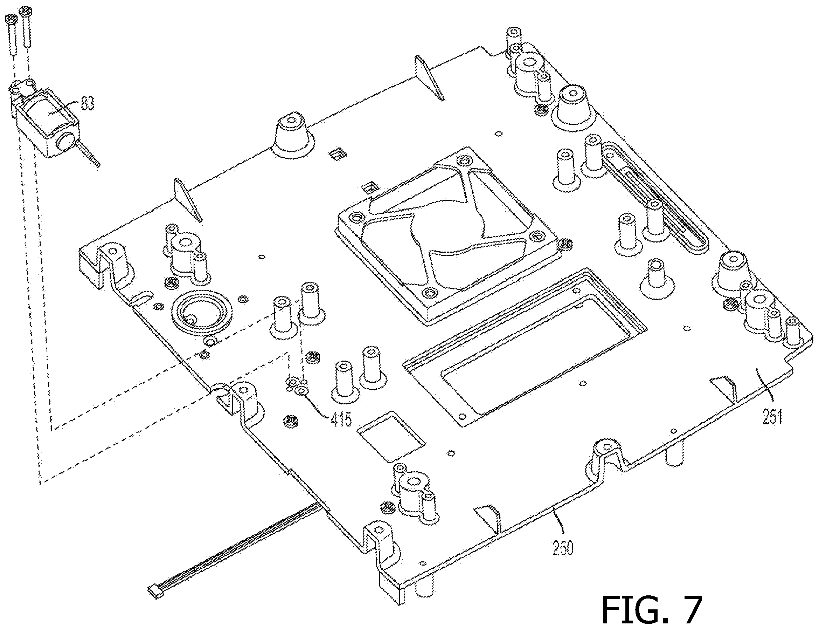

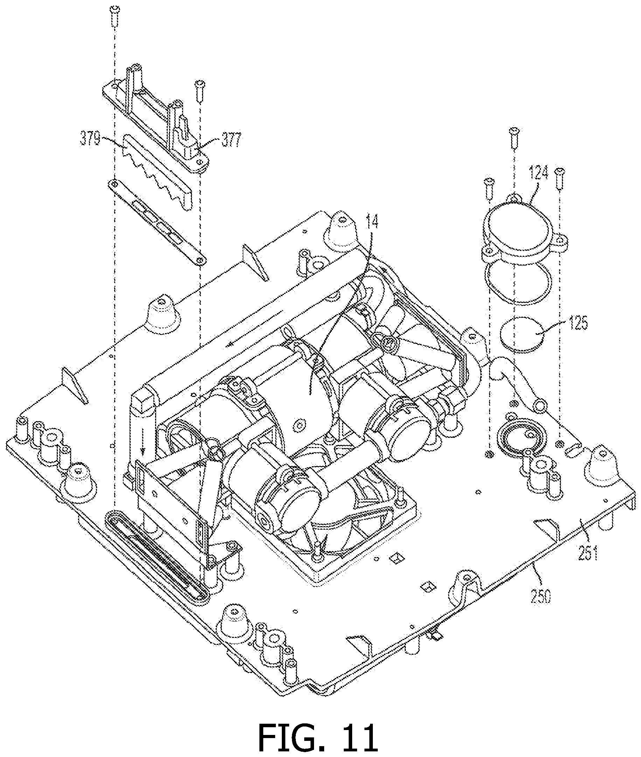

First side surface 251 of support member 250 may also include a muffler attachment portion 411, an air filter attachment portion 413, and an oxygen side balance valve attachment portion 415 that are configured to receive a muffler 377 (FIG. 11), an air filter 124 (FIG. 11) and an oxygen side balance valve 83 (FIG. 7) and to attach muffler 377, air filter 124 and oxygen side balance valve 83 to support member 250. The structure and operation of muffler 377, air filter 124 and oxygen side balance valve 83 will be clear from the discussions below.

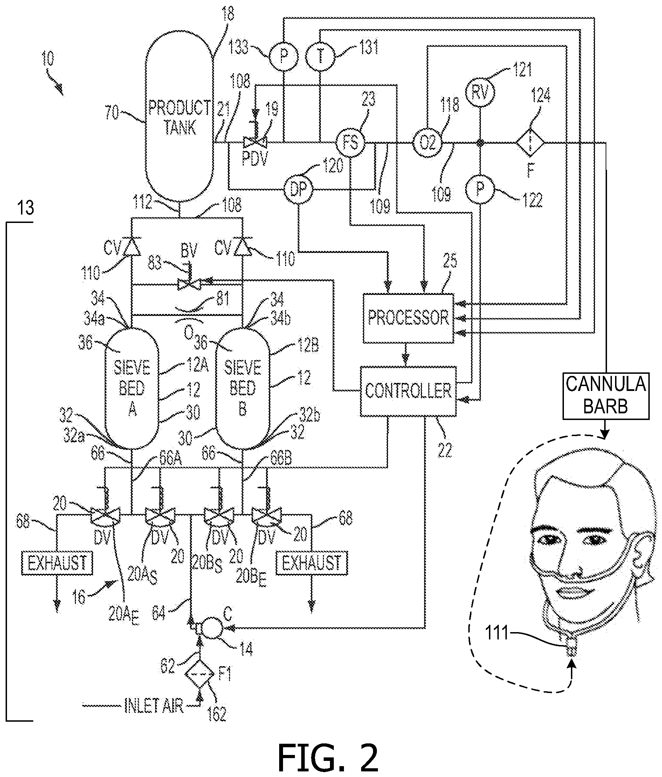

FIG. 4B shows a perspective view of second side surface 253 of support member 250. Air manifold 16 (as shown in FIGS. 2, 3 and 4B) may define a plurality of passages 66-68 therein. Air manifold 16 may include channels 67 that at least partially define compressor outlet passage 64, sieve bed inlet passage 66, and an exhaust passage 68.

Oxygen delivery manifold 102 may be provided for delivering oxygen and/or oxygen-enriched gas from one or more sieve beds 12, to reservoir 18, if present, and/or then to a respiratory circuit 111 for delivery to a user of oxygen concentrator system 10. Oxygen delivery manifold 102 may include channels 67 that at least partially define passages 108, 109 (FIG. 2) for communicating with components related to delivering oxygen and/or oxygen-enriched gas to the subject. In one embodiment, channels 67 of air manifold 16 and oxygen delivery manifold 102 are integrally formed or molded with support member 250.

Air manifold 16 and oxygen delivery manifold 102 may be formed from any engineering grade material, e.g., plastic, such as ABS, polycarbonate or composite materials. Air manifold 16 and oxygen delivery manifold 102 may be formed by injection molding and the like. It is noted that the choice of material for air manifold 16 and oxygen delivery manifold 102, or any other component of oxygen concentrator 10, may substantially alter the total weight of oxygen concentrator system 10. Such a change in weight in turn changes any characteristic ratio of oxygen concentrator system 10 that includes the total weight.

Attachment members 423 on second side surface 253 of support member 250 may be configured to support and/or attach reservoir 18, if present, to second side surface 253 of support member 250. In one embodiment, mounts, straps or supports (not shown) may be used to secure reservoir 18 to oxygen concentrator system 10. For example, such mounts, straps or supports may pass through holes 425 of attachment member 423 to secure reservoir 18 to oxygen concentrator system 10.

Attachment members 427 on second side surface 253 of support member 250 are configured to both support and attach controller 22 to second side surface 253 of support member 250. In one embodiment, attachment members 427 and 423 are integrally formed or molded with support member 250.

Second side surface 253 of support member 250 may include cutout portion that allows a tubular passage member (not shown) to pass through. For example, the tubular passage member is configured to guide or direct oxygen from air filter 124 to an overpressure relief valve 121, as will be described in detail below.

Recess 421 on second side surface 253 is configured to receive an exhaust fan (not shown) therein. The exhaust fan is configured to direct exhaust air (generally concentrated nitrogen) from exhaust passage 68 towards controller 22 or other electronics within oxygen concentrator system 10, e.g., for cooling the electronics.

In one embodiment, attachment members 393, 395, 397, 419, 423 and 427, first compressed air passage member 407, oxygen passage members 399, and second compressed air passage members 403 are all formed of the same material as the rest of support member 250. In some embodiments, air manifold 16 and oxygen delivery manifold 102 are formed of the same material as the rest of support member 250.

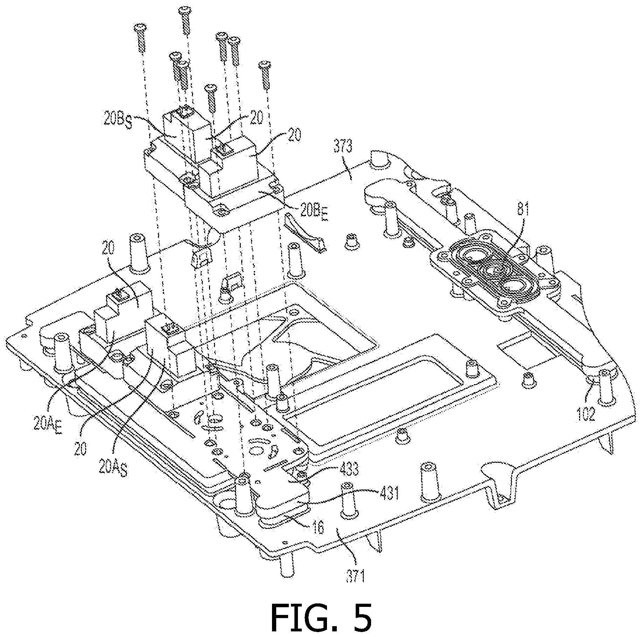

Oxygen concentrator system 10 may include an air manifold cover member 431 configured to cooperate with plurality of channels 67 of air manifold 16 to define passages 64-68 of air manifold 16. That is, air manifold cover member 431 may be configured to interlock with channels 67 (of air manifold cover member 431) of support member 250 to define passages 64-68. In one embodiment, as shown in and explained with respect to FIG. 5, an upper surface 433 of air manifold cover member 431 may be configured to support a set of air control valves 20 (FIG. 5) thereon.

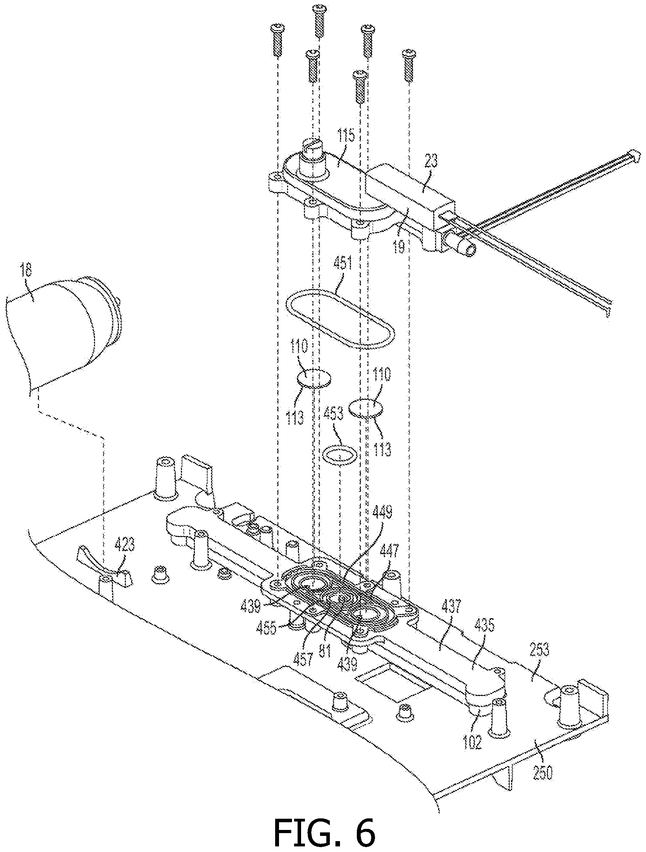

Oxygen concentrator system 10 may include an oxygen delivery manifold cover member 435 configured to cooperate with plurality of channels 67 of oxygen delivery manifold 102 to define passage 108. That is, oxygen delivery manifold cover member 435 may be configured to interlock with channels 67 (of oxygen delivery manifold 102) of support member 250 to define passage 108. In some embodiments, as shown in and explained with respect to FIG. 6, an upper surface 437 of oxygen delivery manifold cover member 435 may be configured to receive a pair of check valves 110 (FIG. 6) therein.

In some embodiments, air manifold cover member 431 and oxygen delivery manifold cover member 435 may be formed of the same material as the rest of support member 250.

As shown in FIGS. 2 and 5, set of air control valves 20 create one or more flow paths through passages 64-68 within air manifold 16. Air control valves are in fluid communication with air manifold 16. Controller 22 may be coupled to air control valves 20 for selectively opening and closing air control valves 20 to control airflow through air manifold 16. That is, air control valves 20 may be selectively opened and closed to provide flow paths, e.g., from compressor outlet passage 64 to sieve bed inlet passage 66 and/or from sieve bed inlet passage 66 to exhaust passage 68. For example, when a supply air control valve 20A_{S} is open, a flow path may defined from compressor 14, through compressor outlet passage 64 and an air control valve 20A_{S}, into a sieve bed 12A. When an exhaust air control valve 20B_{E} is open, a flow path may be defined from a sieve bed 12B, through a sieve bed inlet passage 66B and an air control valve 20B_{E}, and into exhaust passage 68. As noted above, set of air control valves 20 attached to upper surface 433 of air manifold cover member 431 using fasteners.

FIGS. 2 and 6 illustrate check valves 110. Check valves 110 may simply be pressure-activated valves. Check valves 110 may simply be spring biased valves that open in one direction depending upon the pressure differential across the valve, such as conventional umbrella-type valves. When oxygen delivery manifold 102 is mounted to or adjacent sieve beds 12 and reservoir 18, check valves 110 provide one-way flow paths from one or more sieve beds 12 into an oxygen delivery passage 108. In some embodiments, oxygen delivery passage 108 may communicate directly and continuously with reservoir 18 via an opening 112 (FIG. 2).

In one embodiment, check valves 110 may include check disks 113 received in received in spaces 439 formed on upper surface 437 of oxygen delivery manifold cover member 435 and a check valve cover 115 positioned in covering relation to check disks 113. Check valves 110 may be attached to upper surface 437 of oxygen delivery manifold cover member 435 using fasteners. Check valves 110 may be in fluid communication with oxygen delivery manifold 102. Check valves 110 may also include an O-ring 451 that is configured in sealing engagement with edges 447 of a groove 449 on upper surface 437 of oxygen delivery manifold cover member 435.

As shown in FIGS. 2 and 6, oxygen concentrator system 10 may include a purge orifice 81 (FIGS. 5 and 6), which may provide a passage communicating directly between second ports 34 of the one or more sieve beds 12. Optionally, an O-ring 453 may be configured in sealing engagement with edges 455 of a groove 457 on upper surface 437 of oxygen delivery manifold cover member 435. In some embodiments, purge orifice 81 may remain continuously open, thereby providing a passage for oxygen to pass from one sieve bed 12 to another, e.g., while the one sieve bed 12 is charging and the other is purging. In the illustrated embodiment, as shown in FIG. 2, purge orifice 81 maybe disposed upstream of check valves 110. Additional information on an exemplary purge orifice that may be included in oxygen concentrator system 10 may be found in U.S. Patent No. 7,794,522.

FIGS. 2 and 6 show an oxygen delivery valve 19. Oxygen delivery valve 19 may be a proportional valve that is communicating with reservoir 18, if present, via a delivery line 21. Controller 22 receives inputs from sensors, including but not limited to a pressure sensors 120 or 122, an oxygen sensor 118 and/or a flow sensor 23. Controller is configured to control when oxygen delivery valve 19 is fully open, fully closed, or partially open as well as the degree to which oxygen delivery valve 19 is open based on the received inputs from the sensors. In one embodiment, oxygen delivery valve 19 is an adjustable restriction. For example, oxygen delivery valve 19 is a piezo-electric valve, such as a piezo-electric valve manufactured by Festo (Part or Model Number: VEMR-B-6-13-D6-W4-22X5-R5). The piezo-electric valve generally consumes low-power thereby extending the battery life of oxygen concentrator system 10. It is noted that electrical and/or electronic features and/or options used in oxygen concentrator system 10 may substantially alter the power usage, and thus battery life, of oxygen concentrator system 10. Such a change in turn changes any characteristic ratio of oxygen concentrator system 10 that includes power usage and/or battery life.

Flow sensor 23 is associated with a delivery line 21 and is configured to measure the instantaneous mass flow of the oxygen passing through delivery line 21 and to provide feed-back to oxygen delivery valve 19. In one embodiment, flow sensor 23 is a mass flow sensor, such as a flow sensor manufactured by Honeywell (Part or Model Number: AWM 92100V) or a flow sensor manufactured by Festo (Part or Model Number 1238841).

FIGS. 2 and 7 show oxygen side balance valve 83. Oxygen side balance valve 83 is configured to balance bed pressures in, e.g., sieve bed 12A and sieve bed 12B. During the pressure cycling of the one or more sieve beds 12, the pressure in sieve bed 12A may be higher than the pressure in sieve bed 12B indicating that the beds are not balanced. In such an instance, oxygen balance valve 83 is operated (opened) to relieve some pressure from sieve bed 12A and provide the pressure to sieve bed 12B, before compressor 14 switches from sieve bed 12A to sieve bed 12B to supply compressed air to sieve bed 12B. Transferring some pressure from sieve bed 12A to sieve bed 12B allows sieve bed 12B be at some intermediate pressure (rather than be at a zero pressure), when compressor starts supplying compressed air to sieve bed 12B. Since oxygen side balance valve 83 allows sieve bed 12B be at some intermediate pressure (rather than be at a zero pressure), oxygen side balance valve 83 maximizes efficiency, e.g., to reduce power consumption of oxygen concentrator system 10. It is noted that electrical and/or electronic features and/or options used in oxygen concentrator system 10 may substantially alter the power consumption, and thus battery life, of oxygen concentrator system 10. Such a change in turn changes any characteristic ratio of oxygen concentrator system 10 that includes power consumption and/or battery life.

Oxygen side balance valve attachment portion 415 on side surface 251 of support member 250 is configured to receive and to attach oxygen side balance valve 83 to support member 250. Oxygen side balance valve 83 may be in fluid communication with oxygen delivery manifold 102.

Inlet air filter 162 may be provided to remove dust or other particles from the ambient air drawn into inlet openings 160 (FIGS. 14 and 15) before it enters compressor 14. As shown in FIG. 8, inlet air filter 162 maybe positioned in recess 159 on first side surface 251 of support member 250 and may be attached to first side surface 251 of the support member any attachment mechanism, such as fasteners.

Compressor 14 may be any device capable of drawing ambient air into oxygen concentrator system 10 and compressing the air to one or more desired pressures for delivery to one or more sieve beds 12. In one embodiment, compressor 14 is a multiple headed device that includes a motor, a cam assembly coupled to the motor, drive shafts or rods coupled to the cam assembly, and a plurality of diaphragm assemblies or heads coupled to the drive shafts. Additional information on an exemplary compressor that may be included in oxygen concentrator system 10 maybe found in U.S. Patent No. 7,794,522.

As shown in FIG. 9, compressor 14 may be positioned on first side surface 251 of support member 250 using bracket members 165. Optionally, mounting inserts (e.g., foam) 171 may be used with bracket member 165 to provide proper adequate support and damping for compressor 14. Mounting inserts 171A may also be placed between compressor 14 and side surface 251 to provide proper adequate support and noise damping for compressor 14. Compressor 14 may be secured to first side surface 251 of support member 250 using a spring lock assembly 169 having a plurality of springs 167.

FIG. 10 shows a compressor inlet passage 62. Passage members 62A-62C are configured to direct or guide filtered air from an output end 62D of inlet filter 162 to an input (not shown) of compressor 14. Passage members 62A-62C may be connected to each other, to output end 62D of inlet filter 162 and to an input of compressor 14 such that air travels from output end 62D of inlet filter 162, successively through passage members 62A-62C and into an input of compressor 14. Passage members 62A-62C maybe connected to each other, to output end 62D of inlet filter 162 and to an input of compressor 14 using cable ties 463, tee joints 459, clamps 461 and/or any other connection mechanisms. Arrows (FIG. 10) show the flow direction through passage members 62A-62C.

FIG. 10 also shows passage members 14A-C configured to direct compressed air from output end 14D of compressor 14 to compressor outlet passage 64 located in air manifold 16. Passage members 14A-C and 407 may be connected to each other and to output end 14D of compressor 14 such that compressed air travels from output end 14D of compressor 14, successively through passage members 14A-C and 407 and into compressor outlet passage 64 (disposed on second side surface 253 of support member 250) of air manifold 16. Passage members 14A-C and 407 may be connected to each other and to output end 14D of compressor 14 using cable ties, tee joints 459, clamps 461 and/or any other connection mechanisms. Arrows (FIGS. 10 and 11) show the flow direction through passage members 14A-C and 407. In one embodiment, passage member 14A and 62B have bent configurations for space efficiency. It is noted that features and/or options regarding space efficiency used in oxygen concentrator system 10 may substantially alter the total volume of oxygen concentrator system 10. Such a change in turn changes any characteristic ratio of oxygen concentrator system 10 that includes the total volume.

FIG. 11 shows a muffler 377 attached to first side surface 251 of support member 250. Muffler 377 with a baffle 379 maybe configured for muffling the noise of compressor 14. It is noted that features and/or options related to sound produced during operation of oxygen concentrator system 10 may substantially alter the noise level of oxygen concentrator system 10.

As shown in FIG. 11, air filter 124 may be mounted to or adjacent oxygen delivery manifold 102, and may include any a conventional filter media 125 for removing undesired particles from oxygen being delivered to the user. Air filter 124 may be attached to first side surface 251 of support member 250 using any attachment mechanism, such as fasteners. Oxygen delivered from oxygen sensor 118 (FIGS. 14 and 15) may pass through an air filter 124 and be delivered to the user.

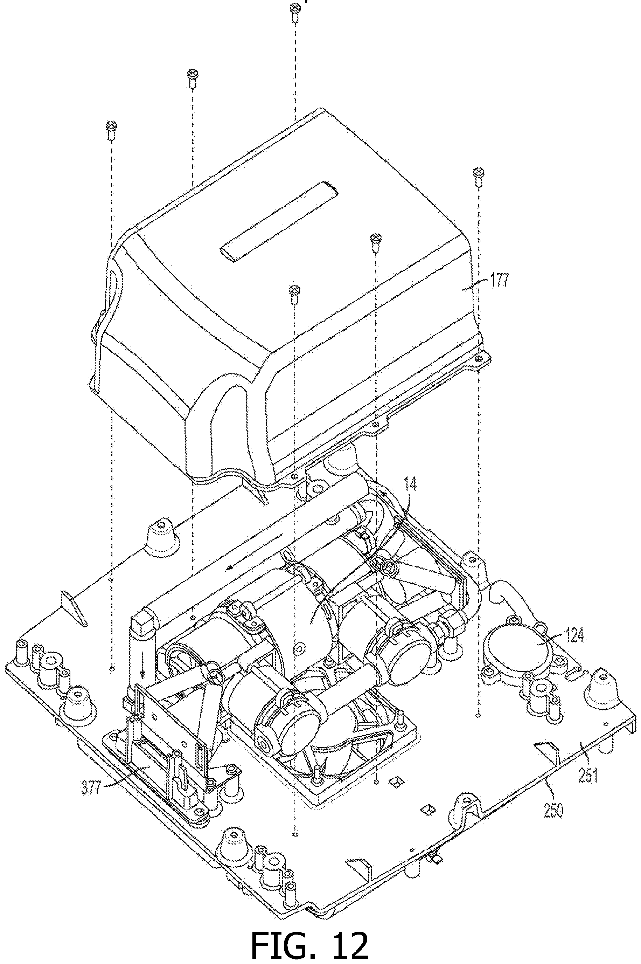

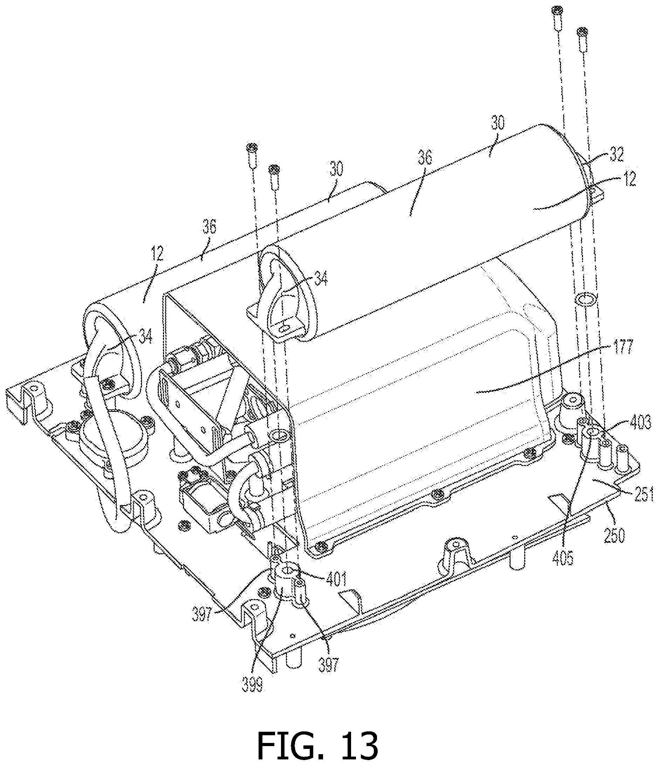

Also, in order to reduce the noise level of compressor 14, a sound shield 177 (as shown in FIGS. 12 and 13) may be formed around compressor 14 to absorb noise generated by the compressor 14. Sound shield 177 is attached to first side surface 251 using fasteners. As shown in FIG. 12, compressor 14, input air filter 162 and sound shield 177 are located on side surface 251 of support member 250. In one embodiment, sound shield 177 is made from (light weight) polypropylene material. In another embodiment, sound shield 177 is made from other plastic or composite materials.

One or more sieve beds 12 maybe configured to absorb nitrogen from air. Each sieve bed 12 may include an outer casing 30, e.g., in the shape of an elongate hollow cylinder, including first port 32 and second port 34. Outer casing 30 may be formed from substantially rigid material, e.g., plastic, such as acrylonitrile butadiene styrene ("ABS"), polycarbonate, and the like, metal, such as aluminum, or composite materials. Outer casing 30 may have any desired shape that may depend upon spatial, performance, and/or structural criteria. For example, outer casing 30 may have a round cylindrical shape, an elliptical, square, rectangular, or other regular or irregular polygonal shaped cross-section.

One or more sieve beds 12 may be attached to first side surface 251 of support member 250 using fasteners installed through holes in attachment members 397 of support member 250. In one embodiment, one or more sieve beds 12 may be attached to support member 250 on both sides of sound shield 177. Each sieve bed 12 maybe attached to support member 250 both at its top and bottom portions.

Oxygen from second ports 34 of the one or more sieve beds 12 enters oxygen delivery manifold 102 through openings 401 of oxygen passage members 399. Compressed air from sieve bed inlet passage 66 of air manifold 16 may enter first ports 32 of the one or more sieve beds 12 through openings 405 of second compressed air passage members 403.

Outer casing 30 may be at least partially filled with filtration media or sieve material 36 to provide one or more sieve beds 12 capable of adsorbing nitrogen from air delivered under pressure. To hold sieve material 36 within casing 30, one or more sieve beds 12 may include discs or plates (not shown) adjacent each of first ports and second ports 32, 34 of casing 30. The plates may be spaced apart from one another to define a desired volume between the plates and within casing 30. The plates may include one or more openings or pores (not shown) therethrough to allow airflow through the plates. Generally, one or more sieve beds 12 may be filled such that there are no substantial voids in sieve material 36, e.g., such that sieve material 36 is substantially packed between the plates. Additional information on exemplary plates that may be included in oxygen concentrator system 10 may be found in U.S. Patent No. 7,794,522.

Sieve material 36 may include one or more known materials capable of adsorbing nitrogen from pressurized ambient air, thereby allowing oxygen to be bled off or otherwise evacuated from sieve bed 12. Exemplary sieve materials that may be used include synthetic zeolite, LiX, and the like, such as UOP Oxysiv 5, 5A, Oxysiv MDX, or Zeochem Z10-06. It may be desirable to provide multiple layers of sieve material 36 within sieve bed 12. For example, it may be desirable to provide sieve material with different properties in layers between first port 32 and second port 34.

Although two sieve beds 12 are shown in FIG. 1, it will be appreciated that one or more sieve beds may be provided, e.g., depending upon the desired weight, performance efficiency, and the like. It is noted that the material used in the one or more sieve beds 12, as well as the number of sieved beds used, may substantially alter the total weight of oxygen concentrator system 10. Such a change in weight in turn changes any characteristic ratio of oxygen concentrator system 10 that includes the total weight. Additional information on exemplary sieve beds and/or sieve materials that may be included in oxygen concentrator system 10 may be found in U.S. Patent Nos. 4,859,217 and 7,794,522.

Reservoir 18, if present, may be in communication with second ports 34 of the one or more sieve beds 12. Reservoir 18 may include an elongate tubular casing for storing oxygen-enriched gas exiting from second ports 34 of the one or more sieve beds 12. The casing of reservoir 18 may be formed from plastic, such as ABS, polycarbonate, and the like, metal, such as aluminum, or composite materials, similar to the other components of oxygen concentrator system 10 described herein.

In a further alternative, oxygen concentrator system 10 may include multiple reservoirs (not shown) that may be provided at one or more locations within oxygen concentrator system 10, e.g., placed in different locations where space is available, yet minimizing the overall dimensions and/or volume of oxygen concentrator system 10. The reservoirs may be connected to one another via one or more flexible tubes (not shown) and/or via oxygen delivery manifold 102 to allow oxygen to be delivered to and withdrawn from the reservoirs. Optionally, in this alternative, one or more valves may be provided for controlling flow of oxygen into and out of the reservoirs.

In addition or alternatively, oxygen concentrator system 10 may include one or more flexible reservoirs, e.g., bags or other containers that may expand or contract as oxygen is delivered into or out of them. The reservoirs may have predetermined shapes as they expand or may expand elastically to fill available space within oxygen concentrator system 10. Optionally, one or more rigid reservoirs may be provided that communicate with one or more flexible reservoirs (not shown), e.g., to conserve space and/or volume within oxygen concentrator system 10. In further alternatives, one or more reservoirs may be provided as portions of one or both of air manifold 16 and oxygen delivery manifold 102, rather than as a separate component, thus further conversing space and/or volume within oxygen concentrator system 10.

As shown in FIGS. 2 and 14, oxygen sensor 118 may also be mounted to and/or below oxygen delivery manifold 102. Oxygen sensor 118 may be capable of measuring the purity of oxygen passing therethrough, e.g., an ultrasonic sensor that measures the speed of sound of the gas passing through oxygen sensor 118, such as those made by Douglas Scientific of Shawnee, Kansas. Alternatively, oxygen sensor 118 may be a ceramic or side-stream sensor.

Oxygen sensor 118 may be coupled to a processor 25 and may generate electrical signals proportional to the purity that may be processed by processor 25 and used by controller 22 to change operation of oxygen concentrator system 10. Because the accuracy of oxygen sensor 118 may be affected by airflow therethrough, it may be desirable to sample the purity signals during no flow conditions, e.g., when oxygen delivery valve 19 is closed.

**5** As shown in FIGS. 2 and 14, oxygen concentrator system 10 may also include an overpressure relief valve 121 pneumatically coupled into delivery line 21 to serve as a protection device for an inhalation sensor 122. Overpressure relief valve 121 allows for the use of a single supply or delivery line to be used for both pulse and continuous flow delivery from oxygen concentrator system 10. Using a single supply or delivery line for multiple modes of operation may obviate the need for at least some of the separate and discrete components typically used to support multiple modes of operation, which may, in turn, reduce the total weight and/or volume of oxygen concentrator system 10. Overpressure relief valve 121 may be set to a level below an operational proof pressure of inhalation sensor 122. If the supply circuit attempts to exceed this proof pressure, due to kinked tubing or otherwise, overpressure relief valve 121 is configured to open and maintain the pressure in the delivery circuit below a level at which inhalation sensor 122 would be damaged. An exemplary overpressure relief valve that may be included in oxygen concentrator system 10 may be found in US provisional patent application no. 61/533,912, filed September 13 2011.

As shown in FIGS. 2 and 14, a pressure sensor 120 may also be mounted to and/or below the oxygen delivery manifold 102 such that ports of pressure sensor 120 may measure a pressure difference between passages 108, 109, and consequently across oxygen delivery valve 19. Optionally, pressure sensor 120 may be used to obtain reservoir pressure. For example, when oxygen delivery valve 19 is closed, pressure upstream of oxygen delivery valve 19 may correspond substantially to the pressure within reservoir 18.

Pressure sensor 120 may be coupled to processor 25, e.g., to provide signals that may be processed by processor 25 to determine the pressure differential across oxygen delivery valve 19. Controller 22 may use this pressure differential to determine a flow rate of the oxygen being delivered from oxygen concentrator system 10 or other parameters of oxygen being delivered. Controller 22 may change the frequency and/or duration that oxygen delivery valve 19 is open based upon the resulting flow rates, e.g., based upon one or more feedback parameters.

As shown in FIG. 2, oxygen concentrator system 10 may include an oxygen gas temperature sensor 131, such as a thermistor, a thermocouple, or any other temperature sensor and a local pressure sensor 133, such as a barometric pressure sensor manufactured by Freescale (Part or Model Number: MPXM2102A). Oxygen gas temperature sensor 131 is configured to measure the temperature of the oxygen passing through delivery line 21, while local pressure sensor 133 is configured to measure the local ambient pressure.

5 The measured oxygen temperature and the measured local ambient pressure are sent to a processor 25. Processor 25 may be configured to use this oxygen temperature measurement from temperature sensor 131 and the local ambient pressure measurement from local pressure sensor 133 along with the mass flow rate measurement obtained from flow sensor 23 to obtain a volumetric flow rate measurement.

In the illustrated embodiment, as shown in FIG. 4, oxygen gas temperature sensor 131 and local pressure sensor 133 are positioned upstream of flow sensor 23. In another embodiment, oxygen gas temperature sensor 131 and local pressure sensor 133 may be positioned downstream (though still in the vicinity) of flow sensor 23.

Pressure sensor 122 may be coupled to oxygen delivery manifold 102. Pressure sensor 122 may be a piezo resistive pressure sensor capable of measuring absolute pressure. Pressure sensor 122 provides a pressure reading that may be used to detect when a user is beginning to inhale. Exemplary transducers that may be used include the Honeywell Microswitch 24PC01SMT Transducer, the Sensym SX01, Motorola MOX, or others made by All Sensors. Because pressure sensor 122 may be exposed to the full system pressure of oxygen concentrator system 10, it may be desirable for the over-pressure rating of pressure sensor 122 to exceed the full system pressure. Pressure sensor 122 may be coupled to processor 25 for providing signals proportional to the pressure detected by pressure sensor 122. Additional information on an exemplary pressure sensor that may be included in oxygen concentrator system 10 may be found in U.S. Patent No. 7,794,522.

In addition, although the components, e.g., oxygen delivery valve 19, pressure sensors 120, 122, 133, flow sensor 23, oxygen sensor 118, oxygen gas temperature sensor 131 and air filter 124 are described in a particular sequence (relative to oxygen flowing through oxygen delivery manifold 102), the sequence of these components may be changed, if desired.

Controller 22 may include one or more hardware components and/or software modules that control one or more aspects of the operation of oxygen concentrator system 10. Controller 22 may be coupled to one or more components of oxygen concentrator system 10, e.g., compressor 14, air control valves 20, and/or oxygen delivery valve 19. Controller 22 may also be coupled to one or more sensing components of oxygen concentrator system 10, e.g., pressure sensors 120, 122, oxygen gas temperature sensor 131, local pressure sensor 133, flow sensor 23 and/or oxygen sensor 118 via processor 25. The components may be coupled by one or more wires or other electrical leads capable of receiving and/or transmitting signals between controller 22 and the components.

Controller 22 may also be coupled to a user interface 320, which may include one or more displays and/or input devices. User interface 320 may be a touch-screen display that may be mounted to oxygen concentrator system 10. User interface 320 may display information regarding parameters related to the operation of oxygen concentrator system 10 and/or allow the user to change the parameters, e.g., turn oxygen concentrator system 10 on and off, change dose setting or desired flow rate, etc. Oxygen concentrator system 10 may include multiple displays and/or input devices, e.g., on/off switches, dials, buttons, and the like. User interface 320 may be coupled to controller 22 by one or more wires and/or other electrical leads (not shown for simplicity), similar to the other components.

Controller 22 may include a single electrical circuit board that includes a plurality of electrical components thereon. These components may include one or more processors, memory, switches, fans, battery chargers, and the like (not shown) mounted to the circuit board. It will be appreciated that controller 22 may be provided as multiple subcontrollers that control different aspects of the operation of oxygen concentrator system 10. For example, a first subcontroller may control operation of compressor 14 and the sequence of opening and closing of air control valves 20, e.g., to charge and purge one or more sieve beds 12 in a desired manner. Additional information on an exemplary first subcontroller that may be included in oxygen concentrator system 10 may be found in U.S. Patent No. 7,794,522.

A second subcontroller may control operation of oxygen delivery valve 19, e.g., to deliver oxygen from reservoir 18 to a user based upon signals received from pressure sensor 120, from flow sensor 23, from oxygen gas temperature sensor 131 and from local pressure sensor 133. The second subcontroller may also receive input instructions from the user and/or display information on user interface 320. In addition, the subcontrollers or other components of controller 22 may share information in a desired manner, as described below. Thus, controller 22 may include one or more components, whose functionality may be interchanged with other components, and controller 22 should not be limited to the specific examples described herein.

Oxygen concentrator system 10 may include one or more power sources, coupled to controller 22, processor 25, compressor 14, air control valves 20, and/or an oxygen delivery valve 23. For example, one or more batteries may be provided that may be mounted or otherwise secured to oxygen concentrator system 10. In one embodiment, one or more batteries 361A (FIG. 1) maybe provided in battery compartment 361 (FIG. 1). Mounts, straps or supports (not shown) maybe used to secure the batteries to oxygen concentrator system 10. Additional information on exemplary batteries that may be included in oxygen concentrator system 10 may be found in U.S. Patent No. 7,794,522.

Controller 22 may control distribution of power from one or more batteries 361A to other components within oxygen concentrator system 10. For example, controller 22 may draw power from one of the one or more batteries 361A until its power is reduced to a predetermined level, whereupon controller 22 may automatically switch to another one of the one or more batteries 361A.

Optionally, oxygen concentrator system 10 may include an adapter such that an external power source, e.g., a conventional AC power source, such as a wall outlet, or a portable AC or DC power source, such as an automotive lighter outlet, a solar panel device, and the like (not shown). Any transformers or other components (also not shown) necessary to convert such external electrical energy such that it may be used by oxygen concentrator system 10 may be provided within oxygen concentrator system 10, in the cables connecting oxygen concentrator system 10 to the external power source, or in the external device itself.

Optionally, controller 22 may direct some electrical energy from external sources back to one or more batteries 361A to recharge them in a conventional manner. Controller 22 may also display the status of the electrical energy of oxygen concentrator system 10, e.g., automatically or upon being prompted via user interface 320, such as the power level of one or more batteries 361A, whether oxygen concentrator system 10 is connected to an external power source, and the like. Controller 22 may include one or more dedicated components for performing one or more of these functions. An exemplary battery management integrated circuit that may be included in controller 22 of oxygen concentrator system 10 may be found in U.S. Patent No. 7,794,522.

Processor 25 of oxygen concentrator system 10 may be configured to receive the signals from one or more sensing components of oxygen concentrator system 10, e.g., flow sensor 23, oxygen gas temperature sensor 131, local pressure sensor 133 and/or pressure sensor 120 to determine a flow of the oxygen-enriched gas in the delivery line over a predetermined period of time, a volume of the oxygen-enriched gas in the delivery line over a predetermined period of time or both based on the received signal.

Oxygen concentrator system 10 may also include a dynamic noise control that is configured to dynamically change an inlet port size or shape of the inlet air filter 162 proportionately for all input/output settings. For example, the higher the volume of air needed the larger the input port size and vice versa. An exemplary dynamic noise control that may be included in oxygen concentrator system 10 may be found in US provisional patent application no. 61/533,864, filed September 13 2011.

The basic operation of oxygen concentrator system 10 will now be described. Generally, operation of oxygen concentrator system 10 has two aspects, concentrating oxygen from ambient air by adsorption within one or more sieve beds 12, and delivering concentrated oxygen to a user, e.g. from reservoir 18. Each aspect of oxygen concentrator system 10 may operate independently of the other, or they may be interrelated, e.g., based upon one or more related parameters.

Oxygen concentrator system 10 may be operated using one or more optional methods, such as those described below, to increase efficiency or other performance characteristics of oxygen concentrator system 10. For example, based upon measurements of pressure and/or flow sensors, the operating conditions of oxygen concentrator system 10 may be adjusted to increase output flow rate and/or pressure, reduce power consumption, and the like.

The aspects of receiving ambient air, filtering the ambient air, compressing the ambient air, and delivering compressed air to air manifold 16 are described by referring FIGS. 2, 10 and 15.

As shown in FIG. 15, ambient air may enter hollow interior 75 of housing 59 through one or more inlet openings 160 (FIGS. 14 and 15) located on bottom wall 69. As noted above, inlet openings 160 are configured to allow the ambient air to pass easily through inlet openings 160, yet preventing large objects from passing therethrough.

Referring to FIGS. 2 and 10, the ambient air in hollow interior 75 may enter inlet air filter 162 through an opening (e.g., located on side 333) of inlet air filter 162. Inlet air filter 62 may be provided before the inlet port of compressor 14 to remove dust or other particles from the ambient air drawn into inlet opening 160 before it enters compressor 14.

Filtered air travels from output end 62D of inlet filter 162, successively through passage members 62A-62C, and into an input of compressor 14. Arrows (FIG. 10) show the flow direction of filtered air through passage members 62A-62C.

Filtered air entering compressor 14 is compressed therein. Compressed air travels from output end 14D of compressor 14, successively through passage members 14A-C and 407 and into compressor outlet passage 64 of air manifold 16. Arrows (FIGS. 10 and 11) show the flow direction through passage members 14A-C and 407. Compressed air may enter compressor outlet passage 64 of air manifold 16 through compressed air passage member 407.

Referring to FIG. 2, 4A, 4B and 5, air control valves 20 are configured to create one or more flow paths through passages 64-68 within air manifold 16. As noted above, air control valves 20 are selectively opened and closed to provide flow paths, e.g., from compressor outlet passage 64 to sieve bed inlet passage 66 and/or from the sieve bed inlet passage 66 to the exhaust passage 68.

The compressed air in sieve bed inlet passage 66 are guided or directed to first ports 32 of the one or more sieve beds 12 via second compressed air passage members 403. The aspect of concentrating oxygen from ambient air by adsorption within one or more sieve beds 12 is explained in great detail in U.S. Patent No. 7,794,522. Exhaust passage 68 communicates with one or more sieve beds 12 to evacuate nitrogen from one or more sieve beds 12.

Concentrated oxygen from second ports 34 of the one or more sieve beds 12 may enter oxygen delivery manifold 102 via oxygen passage members 399. Check valves 110 in oxygen delivery manifold 102 may provide one-way flow paths from second ports 34 of the one or more sieve beds 12 into oxygen delivery passage 108. Concentrated oxygen may be delivered to reservoir 18 via passages 108 of oxygen delivery manifold 102.

With concentrated oxygen stored in reservoir 18, at least in embodiments that include reservoir 18, oxygen concentrator system 10 may be used to deliver concentrated oxygen to a user. As described above, controller 22 may be coupled to oxygen delivery valve 19 for opening and closing oxygen delivery valve 19 to deliver oxygen from reservoir 18 to a user of oxygen concentrator system 10.

In one embodiment, controller 22 may periodically open oxygen delivery valve 19 for predetermined "pulses." During pulse delivery, a "bolus" of oxygen is delivered to the user, i.e., oxygen delivery valve 19 is opened for a predetermined pulse duration, and thereafter closed until the next bolus is to be delivered. Alternatively, controller 22 may open oxygen delivery valve 19 for continuous delivery, e.g., throttling oxygen delivery valve 19 to adjust the flow rate to the user. In a further alternative, controller 22 may periodically open and throttle oxygen delivery valve 19 for a predetermined time to vary the volume of the bolus delivered.

The aspect of controlling opening and closing oxygen delivery valve 19 to deliver the oxygen-enriched gas from reservoir 18 to a user using flow sensor 23 and/or pressure sensor 120 is explained in detail in US provisional patent application no. 61/533,871, filed September 13, 2011.

The components and features of oxygen concentrator system 10 are configured to reduce the total weight and/or volume, while maintaining performance and efficiency in various modes of operation. The O₂ output of oxygen concentrator system 10 is determined as the maximum continuous oxygen output of the oxygen concentrator system using a given configuration of one or more batteries 361A as sole power supply. For example, the O₂ output using one battery may be greater than about 1.8 lpm, greater than about 2.0 lpm, greater than about 2.2 lpm, about 1.86 lpm, and/or other values. A ratio R_{OW} may be determined as: R_{OW} = (O₂ output)/total weight of the oxygen concentrator system. The total weight of oxygen concentrator system 10 may depend on the given configuration of one or more batteries 361A as the sole power supply. For example, the total weight using one battery may be less than about 9.0 lbs, less than about 10.0 lbs, less than about 10.5 lbs, and/or other weights. The total weight using two batteries may be less than about 11.0 lbs, less than about 11.5 lbs, less than about 12 lbs, and/or other weights.

Oxygen concentrator system 10 maybe configured such that the ratio R_{OW} is greater than about 0.16 lpm/lbs, greater than about 0.18 lpm/lbs, greater than about 0.2 lpm/lbs, greater than about 0.22 lpm/lbs, and/or other ratios for a two-battery configuration. Oxygen concentrator system 10 may be configured such that the ratio R_{OW} is greater than about 0.17 lpm/lbs, greater than about 0.19 lpm/lbs, greater than about 0.21 lpm/lbs, or greater than about 0.24 lpm/lbs for a one-battery configuration.

Oxygen concentrator system 10 may be configured such that the total volume is less than about 625 cubic inches, less than about 640 cubic inches, less than about 675 cubic inches, about 636 cubic inches, and/or other volumes. In some embodiments, the cubic shape dimensions of oxygen concentrator system 10 may be 11.29" * 10.74" * 6.74" (i.e. height * width * thickness). A ratio R_{OV} maybe determined as: R_{OV}= (O₂ output)/total volume, and wherein the R_{OV} for the oxygen concentrator may be not less than about 2.9 ^{∗} 10⁻³ lpm/cubic inch, not less than about 3.2 ^{∗} 10⁻³ lpm/cubic inch, not less than about 3.4 ^{∗} 10⁻³ lpm/cubic inch, not less than about 3.0 ^{∗} 10⁻³ lpm/cubic inch, and/or other ratios.

The maximum duration of oxygen concentrator system 10 operating under operating conditions of maximum continuous oxygen output using a single charge of a single battery as the sole power supply may be referred to as the single-battery battery life of oxygen concentrator system 10. In some embodiments, oxygen concentrator system 10 is configured such that a battery can be fully charged in less than 4 hours when plugged into a 120V AC outlet. The maximum duration of oxygen concentrator system 10 operating under operating conditions of maximum continuous oxygen output using a single charge of two batteries as the sole power supply may be referred to as the dual-battery battery life of oxygen concentrator system 10. Oxygen concentrator system 10 may be configured such that the single-battery battery life is greater than about 30 minutes, greater than about 45 minutes, greater than about 1 hour, and/or other durations, whereas the dual-battery battery life is greater than about 1 hour, greater than about 1.5 hours.

A ratio R_{OD} maybe determined as: R_{OD}=(O₂ output ^{∗} duration), where duration is the battery life of oxygen concentrator 10. Oxygen concentrator system 10 may be configured such that R_{OD} is greater than about 1.0 (lpm-hr) or 56 1, greater than about 1.5 (lpm-hr) or 90 1, greater than about 1.8 (lpm-hr) or 110 l, greater than about 2.2 (lpm-hr) or 130 l, and/or other ratios for single-battery configurations. Oxygen concentrator system 10 may be configured such that and R_{OD} is greater than about 1.8 (lpm-hr) or 110 l, greater than about 3.0 (lpm-hr), greater than about 3.6 (lpm-hr) or 220 1, greater than about 4.4 (lpm-hr), and/or other ratios for dual-battery configurations.

A ratio R_{ODW} may be determined as: R_{ODW}=(O₂ output ^{∗} duration)/total weight, where duration is the battery life of oxygen concentrator 10. Oxygen concentrator system 10 may be configured such that R_{ODW} is greater than about 0.1 (lpm-hr/lbs) or 5.6 l/lbs, greater than about 0.15 (lpm-hr/lbs) or 9 l/lbs, greater than about 0.22 (lpm-hr/lbs) or 13 l/lbs, and/or other ratios for single-battery configurations. Oxygen concentrator system 10 may be configured such that and R_{ODW} is greater than about 0.16 (lpm-hr/lbs) or 9.7 l/lbs, greater than about 0.22 (lpm-hr/lbs) or 13 l/lbs, greater than about 0.28 (lpm-hr/lbs) or 17 l/lbs, greater than about 0.38 (lpm-hr/lbs) or 23 l/lbs, and/or other ratios for dual-battery configurations.

A ratio R_{ODV} may be determined as: R_{ODV}=(O₂ output ^{∗} duration)/total volume. Oxygen concentrator system 10 may be configured such that R_{ODV} is greater than about 2.4 ^{∗} 10⁻³ (lpm-hr)/cubic inch or 0.14 l/cubic inch, greater than about 2.7 ^{∗} 10⁻³ (lpm-hr)/cubic inch, greater than about 3.0 ^{∗} 10⁻³ (lpm-hr)/cubic inch, greater than about 1.5 ^{∗} 10⁻³ (lpm-hr)/cubic inch, and/or other ratios for single-battery configurations. Oxygen concentrator system 10 may be configured such that R_{ODV} is greater than about 4.5 ^{∗} 10⁻³ (lpm-hr)/cubic inch or 0.27 l/cubic inch, greater than about 5.3 ^{∗} 10⁻³ (lpm-hr)/cubic inch, greater than about 5.8 ^{∗} 10⁻³ (lpm-hr)/cubic inch, greater than about 2.9 ^{∗} 10⁻³ (lpm-hr)/cubic inch, and/or other ratios for dual-battery configurations.

The present disclosure also provides a method of manufacturing an oxygen concentrator system. The method includes forming support member 250 configured to support one or more of compressor 14, sieve beds 12 and reservoir 18, integrally forming air manifold 16 at a lower portion of support member 250, and integrally forming oxygen delivery manifold 102 at upper portion 371 of support member 250. The method further may include integrally forming first compressed air passage member 407, second compressed air passage members 403 and oxygen passage members 399 with support member 250 and integrally forming attachment members 393, 395, 397, 419 and 427 with support member 250.

The method also may include attaching compressor 14 and one or more sieve beds 12 to first side surface 251 of support member 250, attaching reservoir 18 to second side surface 253 of support member 250, attaching air manifold cover member 431 to air manifold 16 on support member 250, and attaching oxygen delivery manifold cover member 435 to oxygen delivery manifold 102 on support member 250. Attaching other components of oxygen concentrator system 10, including but not limited to valves, controller, processor, sensors, sound shield, muffler, tubing or passage members, and filters to support member 250.

Support member 250 with components of oxygen concentrator system 10 attached thereon is connected to one of two mating housing members 59A and 59B. The one mating housing member and support member 250 may then be connected to other of mating housing members 59A and 59B.

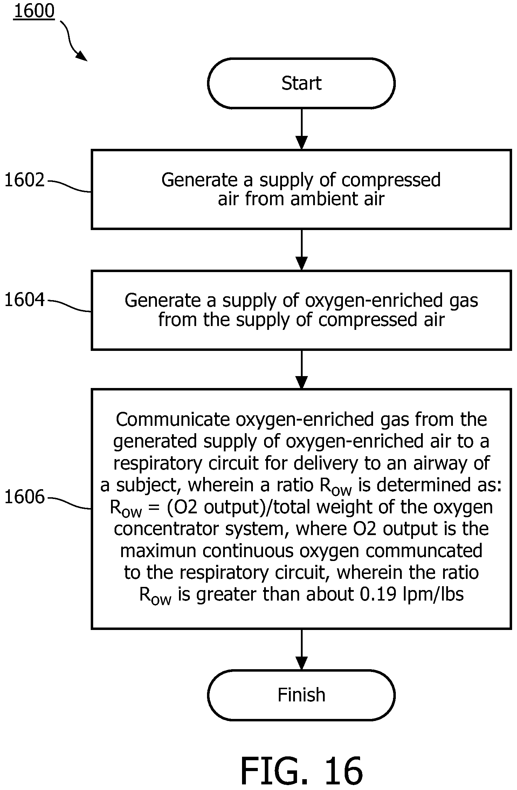

FIG. 16 illustrates a method 1600 for concentrating oxygen using an oxygen concentrator system. The operations of method 1600 presented below are intended to be illustrative. In some embodiments, method 1600 may be accomplished with one or more additional operations not described, and/or without one or more of the operations discussed. Additionally, the order in which the operations of method 1600 are illustrated in FIG. 16 and described below is not intended to be limiting. In some embodiments, method 1600 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 1600 in response to instructions stored electronically on an electronic storage medium. The one or more processing devices may include one or more devices configured through hardware, firmware, and/or software to be specifically designed for execution of one or more of the operations of method 1600.

At an operation 1602, a supply of compressed air is generated from ambient air. In one embodiment, operation 1602 is performed using a compressor similar to or substantially the same as compressor 14 (shown in FIG. 2 and described above).

At an operation 1604, a supply of oxygen-enriched gas is generated from the supply of compressed air. In one embodiment, operation 1604 is performed by one or more sieve beds similar to or substantially the same as one or more sieve beds 12 (shown in FIG. 2 and described above).

At an operation 1606, oxygen-enriched gas is communicated from the generated supply of oxygen-enriched gas to a respiratory circuit for delivery to an airway of a subject. A ratio R_{OW} of the oxygen concentrator system is determined as: R_{OW} = (O₂ output)/total weight of the oxygen concentrator system, where O₂ output is the maximum continuous oxygen communicated to the respiratory circuit. In one embodiment, operation 1606 is performed by an oxygen delivery subsystem similar to or substantially the same as oxygen delivery subsystem 11 (shown in FIG. 3 and described above).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the embodiments have been described in detail for the purpose of illustration based on what is currently considered to be most practical and preferred, it is to be understood that such detail is solely for that purpose and does not impose any limits, but, on the contrary, the disclosure is intended to cover modifications and equivalent arrangements that are within the scope of the appended claims. For example, it is to be understood that, to the extent possible, one or more features of any embodiment are contemplated to be combined with one or more features of any other embodiment.

## Claims

1. An oxygen concentrator system (10), comprising:
an oxygen concentration subsystem (13) configured to implement adsorption to generate a supply of oxygen-enriched gas by means of a first sieve bed (12A) and a second sieve bed (12B), the oxygen concentration subsystem (13) further comprising:
a compressor (14) configured to deliver pressurized air to said sieve beds via a first port (32) of the sieve beds;
an air manifold (16) providing a plurality of channels therein that at least partially define inlet air passages (64, 65, 66, 67, 68) communicating between the compressor and the first port(32); and
air control valves (20) configured to create one or more flow paths through the inlet air passages through selective opening and closing of the air control valves, thereby to control airflow through the air manifold; and
an oxygen delivery subsystem (11) configured to communicate oxygen-enriched gas from the oxygen concentration subsystem (13) to a respiratory circuit, the oxygen delivery subsystem comprising:
an oxygen delivery manifold (102) configured to receive the oxygen-enriched gas from a second port (34) of the sieve beds, and to deliver the oxygen-enriched gas to an airway of a subject,
wherein the oxygen concentrator system further comprises:
an oxygen side balance valve (83) in fluidic communication between the one or more sieve beds and configured to balance pressures of the sieve beds by enabling transfer of pressure from one of the respective sieve beds which is pressurized to the other sieve bed having an internal pressure lower than the pressure of the pressurized sieve bed, such that pressure transferred from the pressurized sieve bed to the other sieve bed (12B) allows said other sieve bed to be at an intermediate pressure when the compressor starts supplying compressed air to said other sieve bed;
one or more batteries (361A) configured to act as a sole power supply for the oxygen concentrator system (10), wherein the one or more batteries (361A) has a single-charge battery life of greater than about 1.5 hours during operating conditions of maximum continuous oxygen output;
a housing (59) configured to house the oxygen concentration subsystem (13), the oxygen delivery subsystem (11), the balance valve and the one or more batteries; and
wherein a ratio ROW of the oxygen concentrator system (10) is determined as: ROW = (O2 output)/total weight of the oxygen concentrator system housed within the housing, where O2 output is the maximum continuous oxygen output of the oxygen concentrator system, and wherein the ratio ROW is greater than about 0.19 lpm/lbs (0.42 lpm/kg).

2. The oxygen concentrator system (10) of claim 1 further comprising:
a reservoir (18) configured to store the oxygen-enriched gas; and
a support member (250) configured to support the compressor (14) the sieve beds (12) and the reservoir, wherein air manifold (16) and the oxygen delivery manifold (102) is integrally formed in said support member (250).

3. The oxygen concentrator system (10) of claim 2, further comprising:
an oxygen delivery valve (19), said oxygen delivery valve being in communication with the reservoir (18);
a pressure sensor (120, 122), an oxygen sensor (118) and/or a flow sensor (23); and
a controller (22) configured to control the oxygen delivery valve to be fully open, fully closed or partially open, and a degree to which the delivery valve is open based on inputs received from the pressure sensor, the oxygen sensor and/or the flow sensor, wherein said oxygen delivery valve (19) is a piezo-electric valve (19).

4. The oxygen concentrator system (10) of claim 2 wherein the oxygen delivery manifold is integrally formed with a lower portion (371) of the support member (250).

5. The oxygen concentrator system (10) of claim 1, wherein the oxygen concentrator system has a total weight of less than about 4.54 kg (10 lbs).

6. The oxygen concentrator system (10) of claim 1, wherein the oxygen concentrator system has a total volume less than about 10.5 liters (640 cubic inches).

7. The oxygen concentrator system (10) of claim 1, wherein a ratio ROD of the oxygen concentrator system (10) is determined as: ROD = (O2 output ^{∗} duration), where duration is the operating life of the oxygen concentrator over a single charge of the one or more batteries during operating conditions of maximum continuous oxygen output, and wherein the ROD for the oxygen concentrator is not less than about 110 liters.

8. The oxygen concentrator system (10) of claim 1, wherein a ratio RODW of the oxygen concentrator system (10) is determined as: RODW = (O2 output ^{∗} duration)/total weight of the oxygen concentrator system, where duration is the operating life of the oxygen concentrator over a single charge of the one or more batteries during operating conditions of maximum continuous oxygen output, and wherein the RODW for the oxygen concentrator is not less than about 0.48 (lpm-hr)/kg (0.22 (lpm-hr)/lbs).

9. A method for concentrating oxygen using the oxygen concentrator system according to claim 1, wherein power is supplied to the oxygen concentrator system solely through said one or more batteries (361A) having a single-charge battery life of greater than about 1.5 hours during operating conditions of maximum continuous oxygen output, the method comprising the steps of:
generating a supply of compressed air from ambient air via the concentrator;
generating a supply of oxygen-enriched gas from the supply of compressed air via the sieve beds;
balancing the sieve beds such that said one of the sieve beds is at said intermediary pressure via the balancing valve in fluidic communication with the sieve beds; and
communicating oxygen-enriched gas from the generated supply of oxygen-enriched gas to a respiratory circuit for delivery to an airway of a subject, wherein a ratio ROW is determined as: ROW = (O2 output)/total weight of the oxygen concentrator system, where O2 output is the maximum continuous oxygen communicated to the respiratory circuit, wherein the ratio ROW is greater than about 0.42 lpm/kg (0.19 lpm/lbs).

10. The method of claim 9, wherein the oxygen concentrator system has a total weight of less than about 4.54 kg (10 lbs).

11. The method of claim 9, further comprising supplying power to the oxygen concentrator system solely through one or more batteries, wherein a single-charge battery life of the one or more batteries when the one or more batteries are acting as the sole power supply to the oxygen concentrator system during operating conditions of maximum continuous oxygen output is greater than about 1.5 hours.

12. The method of claim 9, wherein the oxygen concentrator system has a total volume less than about 10.5 liters (640 cubic inches).

13. The method of claim 11, wherein a ratio ROD is determined as: ROD = (O2 output ^{∗} duration), where duration is the operating life of the oxygen concentrator over a single charge of the one or more batteries during operating conditions of maximum continuous oxygen output, and wherein the ROD for the oxygen concentrator is not less than about 110 liters.

14. The method of claim 11, wherein a ratio RODW is determined as: RODW = (O2 output ^{∗} duration)/total weight of the oxygen concentrator system, where duration is the operating life of the oxygen concentrator over a single charge of the one or more batteries during operating conditions of maximum continuous oxygen output, and wherein the RODW for the oxygen concentrator is not less than about or 0.48 (lpm-hr)/kg. (0.22 (lpm-hr)/lbs).

## Patentansprüche

1. Sauerstoffkonzentratorsystem (10), das Folgendes umfasst:
ein Sauerstoffkonzentrations-Subsystem (13), das dazu konfiguriert, eine Adsorption zu implementieren, um eine Zufuhr von sauerstoffangereichertem Gas mittels eines ersten Siebbettes (12A) und eines zweiten Siebbettes (12B) zu erzeugen, wobei das Sauerstoffkonzentrations-Subsystem (13) ferner Folgendes umfasst:
einen Kompressor (14), der dazu konfiguriert ist, Druckluft über eine erste Öffnung (32) der Siebbetten zu den Siebbetten zu liefern;
einen Luftverteiler (16), der mehrere Kanäle darin bereitstellt, die zumindest teilweise die Einlassluftkanäle (64, 65, 66, 67, 68) definieren, die zwischen dem Kompressor und dem ersten Anschluss (32) kommunizieren; und
Luftsteuerventile (20), die dazu konfiguriert sind, einen oder mehrere Strömungswege durch die Einlassluftkanäle durch selektives Öffnen und Schließen der Luftsteuerventile zu erzeugen, wodurch der Luftstrom durch den Luftverteiler gesteuert wird; und
ein Sauerstoffabgabesubsystem (11), das dazu konfiguriert ist, mit Sauerstoff angereichertes Gas vom Sauerstoffkonzentrations-Subsystem (13) an einen Atmungskreislauf zu übertragen, wobei das Sauerstoffabgabe-Subsystem Folgendes umfasst:
einen Sauerstoffzufuhrverteiler (102), der dazu konfiguriert ist, das mit Sauerstoff angereicherte Gas von einer zweiten Öffnung (34) der Siebbetten aufzunehmen und das mit Sauerstoff angereicherte Gas an einen Atemweg eines Subjekts abzugeben;
wobei das Sauerstoffkonzentratorsystem ferner Folgendes umfasst:
ein sauerstoffseitiges Ausgleichsventil (83) in Fluidverbindung zwischen dem einen oder den mehreren Siebbetten und dazu konfiguriert, die Drücke der Siebbetten auszugleichen, indem die Druckübertragung von einem der jeweiligen Siebbetten, das unter Druck gesetzt wird, auf das andere Siebbett mit einem Innenraum Druck niedriger als der Druck des unter Druck stehenden Siebbettes ermöglicht wird, sodass der vom unter Druck stehenden Siebbett auf das andere Siebbett (12B) übertragene Druck es dem anderen Siebbett ermöglicht, einen Zwischendruck zu haben, wenn der Kompressor beginnt, dem anderen Siebbett Druckluft zuzuführen;
eine oder mehrere Batterien (361A), die als alleinige Stromversorgung für das Sauerstoffkonzentratorsystem (10) ausgelegt sind, wobei die eine oder die mehreren Batterien (361A) unter Betriebsbedingungen von eine Batterielebensdauer von mehr als etwa 1,5 Stunden bei maximaler kontinuierlicher Sauerstoffabgabe aufweisen;
ein Gehäuse (59), das dazu ausgelegt ist, das Sauerstoffkonzentrations-Subsystem (13), das Sauerstoffzufuhr-Subsystem (11), das Ausgleichsventil und die eine oder die mehreren Batterien aufzunehmen; und
wobei ein Verhältnis ROW des Sauerstoffkonzentratorsystems (10) bestimmt wird als: ROW = (O2-Abgabe)/Gesamtgewicht des im Gehäuse untergebrachten Sauerstoffkonzentratorsystems, wobei die O2-Abgabe der maximale kontinuierliche Sauerstoffausstoß des Sauerstoffkonzentratorsystems ist und das Verhältnis ROW größer als etwa 0,19 lpm/lbs (0,42 lpm/kg)) ist.

2. Sauerstoffkonzentratorsystem (10) nach Anspruch 1, das ferner Folgendes umfasst:
ein Reservoir (18), das dazu ausgelegt ist, das mit Sauerstoff angereicherte Gas zu speichern; und
ein Stützelement (250), das dazu ausgelegt ist, den Kompressor (14), die Siebbetten (12) und den Vorratsbehälter zu tragen, wobei der Luftverteiler (16) und
der Sauerstoffzufuhrverteiler (102) einstückig in dem Stützelement (250) ausgebildet ist.

3. Sauerstoffkonzentratorsystem (10) nach Anspruch 2, ferner umfassend:
ein Sauerstoffzufuhrventil (19), wobei das Sauerstoffzufuhrventil in Verbindung mit dem Reservoir (18) steht;
einen Drucksensor (120, 122), einen Sauerstoffsensor (118) und/oder einen Durchflusssensor (23); und
eine Steuerung (22), die dazu konfiguriert ist, das Sauerstoffzufuhrventil so zu steuern, dass es vollständig offen, vollständig geschlossen oder teilweise geöffnet ist und zu einem Grad, zu dem das Abgabeventil offen ist, basierend auf Eingaben, die vom Drucksensor, dem Sauerstoffsensor und/oder dem Durchflusssensor empfangen werden, wobei das Sauerstoffzufuhrventil (19) ein piezoelektrisches Ventil (19) ist.

4. Sauerstoffkonzentratorsystem (10) nach Anspruch 2, wobei der Sauerstoffzufuhrverteiler einstückig mit einem unteren Abschnitt (371) des Trägerelements (250) ausgebildet ist.

5. Sauerstoffkonzentratorsystem (10) nach Anspruch 1, wobei das Sauerstoffkonzentratorsystem ein Gesamtgewicht von weniger als etwa 4,54 kg (10 lbs) aufweist.

6. Sauerstoffkonzentratorsystem (10) nach Anspruch 1, wobei das Sauerstoffkonzentratorsystem ein Gesamtvolumen von weniger als etwa 10,5 Litern (640 Kubikzoll) aufweist.

7. Sauerstoffkonzentratorsystem (10) nach Anspruch 1, wobei ein Verhältnis ROD des Sauerstoffkonzentratorsystems (10) bestimmt wird als: ROD = (O2-Abgabe ^{∗} Dauer), wobei die Dauer die Betriebsdauer des Sauerstoffkonzentrators mit einer einzelnen Ladung der einen oder der mehreren Batterien unter Betriebsbedingungen mit maximaler kontinuierlicher Sauerstoffabgabe ist, und wobei das ROD für den Sauerstoffkonzentrator nicht geringer ist als etwa 110 Liter.

8. Sauerstoffkonzentratorsystem (10) nach Anspruch 1, wobei ein Verhältnis RODW des Sauerstoffkonzentratorsystems (10) bestimmt wird als: RODW = (O2-Abgabe ^{∗} Dauer)/Gesamtgewicht des Sauerstoffkonzentratorsystems, wobei die Dauer die Betriebsdauer des Sauerstoffkonzentrators mit einer einzelnen Ladung der einen oder der mehreren Batterien unter Betriebsbedingungen mit maximaler kontinuierlicher Sauerstoffabgabe ist und wobei das RODW für den Sauerstoffkonzentrator nicht weniger als etwa 0,48 (lpm-h)/kg (0,22 (lpm-h)/lbs) beträgt.

9. Verfahren zum Konzentrieren von Sauerstoff unter Verwendung des Sauerstoffkonzentratorsystems nach Anspruch 1, wobei das Sauerstoffkonzentratorsystem nur über die eine oder die mehreren Batterien (361A) mit einer Batterielebensdauer mit einer Ladung von mehr als etwa 1,5 Stunden unter Betriebsbedingungen der maximalen kontinuierlichen Sauerstoffabgabe mit Strom versorgt wird, wobei das Verfahren die Schritte umfasst:
Erzeugung einer Druckluftzufuhr aus Umgebungsluft über den Konzentrator;
Erzeugung einer Versorgung mit sauerstoffangereichertem Gas aus der Zufuhr von
Druckluft über die Siebbetten;
Ausbalancierung der Siebbetten derart, dass eines der Siebbetten über das Ausgleichsventil in Fluidverbindung mit den Siebbetten auf dem Zwischendruck steht; und
Übertragung von sauerstoffangereichertem Gas aus der erzeugten Zufuhr von sauerstoffangereichertem Gas an einen Atemkreislauf zur Abgabe an einen Atemweg eines Subjekts, wobei ein Verhältnis ROW bestimmt wird als: ROW = (O2-Abgabe)/Gesamtgewicht des Sauerstoffkonzentratorsystems, wobei die O2-Abgabe der maximale kontinuierliche Sauerstoff ist, der an den Atemkreislauf übertragen wird, wobei das Verhältnis ROW größer als etwa 0,42 lpm/kg (0,19 lpm/lbs) ist.

10. Verfahren nach Anspruch 9, wobei das Sauerstoffkonzentratorsystem ein Gesamtgewicht von weniger als etwa 4,54 kg (10 lbs) aufweist.

11. Das Verfahren nach Anspruch 9, das ferner die Versorgung des Sauerstoffkonzentratorsystems ausschließlich durch eine oder mehrere Batterien umfasst, wobei die Lebensdauer der einen oder mehreren Batterien mit einer einzigen Ladung, wenn die eine oder mehreren Batterien als einzige Stromversorgung für das Sauerstoffkonzentratorsystem unter Betriebsbedingungen mit maximaler kontinuierlicher Sauerstoffabgabe fungieren, größer als etwa 1,5 Stunden ist.

12. Verfahren nach Anspruch 9, wobei das Sauerstoffkonzentratorsystem ein Gesamtvolumen von weniger als etwa 10,5 Litern (640 Kubikzoll) aufweist.

13. Verfahren nach Anspruch 11, wobei ein Verhältnis ROD bestimmt wird als: ROD = (O2-Abgabe ^{∗} Dauer), wobei die Dauer die Betriebsdauer des Sauerstoffkonzentrators mit einer einzelnen Ladung der einen oder der mehreren Batterien unter Betriebsbedingungen mit maximaler kontinuierlicher Sauerstoff-Abgabe ist, und wobei das ROD für den Sauerstoffkonzentrator nicht geringer ist als etwa 110 Liter.

14. Verfahren nach Anspruch 11, wobei ein Verhältnis RODW bestimmt wird als: RODW = (O2-Abgabe ^{∗} Dauer)/Gesamtgewicht des Sauerstoffkonzentratorsystems, wobei die Dauer die Lebensdauer des Sauerstoffkonzentrators mit einer einzelnen Ladung der einen oder der mehreren Batterien unter Betriebsbedingungen mit maximaler kontinuierlicher Sauerstoffleistung ist und wobei das RODW für den Sauerstoffkonzentrator nicht weniger als ungefähr oder 0,48 (lpm-h)/kg (0,22 (lpm-h)/lbs) beträgt.

## Revendications

1. Concentrateur d'oxygène (10) comprenant:
un sous-système de concentration d'oxygène (13) configuré pour mettre en œuvre l'adsorption afin de générer un apport de gaz enrichi en oxygène au moyen d'un premier lit à tamis (12A) et d'un second lit à tamis (12B), le sous-système de concentration d'oxygène (13) comprenant en outre:
un compresseur (14) configuré pour fournir de l'air pressurisé auxdits lits à tamis via un premier orifice (32) des lits à tamis;
un collecteur d'air (16) doté d'une pluralité de conduits définissant au moins partiellement des passages d'air d'entrée (64, 65, 66, 67, 68) communiquant entre le compresseur et le premier orifice (32); et
des soupapes de régulation d'air (20) configurées pour créer une ou plusieurs voies d'écoulement à travers les passages d'air d'entrée par le biais d'ouverture et de fermeture sélective des soupapes de régulation d'air, pour ainsi réguler le flux d'air à travers le collecteur d'air; et
un sous-système d'alimentation en oxygène (11) configuré pour communiquer le gaz enrichi en oxygène du sous-système de concentration d'oxygène (13) à un circuit respiratoire, le sous-système d'alimentation en oxygène comprenant:
un collecteur d'alimentation en oxygène (102) configuré pour recevoir le gaz enrichi en oxygène d'un second orifice (34) des lits à tamis, et pour amener le gaz enrichi en oxygène à une voie aérienne d'un sujet,
dans lequel le système de concentration d'oxygène comprend en outre:
une vanne d'équilibrage côté oxygène (83) en communication fluidique entre un ou plusieurs lits à tamis et configurée pour équilibrer les pressions des lits à tamis en permettant le transfert de pression de l'un des lits à tamis respectifs pressurisés vers l'autre lit à tamis ayant une pression interne inférieure à la pression du lit à tamis pressurisé, de sorte que la pression transférée du lit à tamis pressurisé vers l'autre lit à tamis (12B) permette audit autre lit à tamis d'être à une pression intermédiaire lorsque le compresseur commence à amener l'air comprimé audit autre lit à tamis;
une ou plusieurs batteries (361A) configurées pour faire office de source d'alimentation électrique unique pour le concentrateur d'oxygène (10), dans lequel une ou plusieurs batteries (361A) ont une autonomie sur une seule charge supérieure à environ 1,5 heures pendant des conditions de fonctionnement de production d'oxygène continue maximale;
un boîtier (59) configuré pour loger le sous-système de concentration d'oxygène (13), le sous-système d'alimentation en oxygène (11), la vanne d'équilibrage et une ou plusieurs batteries; et
dans lequel un ratio ROW du concentrateur d'oxygène (10) est déterminé en tant que: ROW = (production d'O2) /poids total du concentrateur d'oxygène logé dans le boîtier, où la production d'O2 est la production d'oxygène continue maximale du concentrateur d'oxygène, et dans lequel le ratio ROW est supérieur à environ 0,19 lpm/lbs (0,42 lpm/kg).

2. Concentrateur d'oxygène (10) selon la revendication 1, comprenant en outre:
un réservoir (18) configuré pour stocker le gaz enrichi en oxygène; et
un élément de support (250) configuré pour supporter le compresseur (14), les lits à tamis (12) et le réservoir, dans lequel le collecteur d'air (16) et le collecteur d'alimentation en oxygène (102) sont intégralement formés dans ledit élément de support (250) .

3. Concentrateur d'oxygène (10) selon la revendication 2, comprenant en outre:
une vanne d'alimentation en oxygène (19), ladite vanne d'alimentation en oxygène communiquant avec le réservoir (18);
un capteur de pression (120, 122), un capteur d'oxygène (118) et/ou un capteur de débit (23); et
un régulateur (22) configuré pour réguler la vanne d'alimentation d'oxygène de façon qu'elle soit entièrement ouverte, entièrement fermée ou partiellement ouverte, et un degré dans lequel la vanne d'alimentation est ouverte en fonction des données reçues du capteur de pression, du capteur d'oxygène et/ou du capteur de débit, dans lequel ladite vanne d'alimentation en oxygène (19) est une vanne piézo-électrique (19).

4. Concentrateur d'oxygène (10) selon la revendication 2 dans lequel le collecteur d'alimentation en oxygène est intégralement formé avec une partie inférieure (371) de l'élément de support (250).

5. Concentrateur d'oxygène (10) selon la revendication 1, dans lequel le concentrateur d'oxygène a un poids total inférieur à environ 4,54 kg (10 lbs).

6. Concentrateur d'oxygène (10) selon la revendication 1, dans lequel le concentrateur d'oxygène a un volume total inférieur à environ 10,5 litres (640 pouces cubiques).

7. Concentrateur d'oxygène (10) selon la revendication 1, dans lequel un ratio ROD du concentrateur d'oxygène (10) est déterminé comme: ROD = (durée de production^{∗} d'O2), où la durée représente la durée de vie du concentrateur d'oxygène sur une seule charge d'une ou de plusieurs batteries durant des conditions de fonctionnement de production d'oxygène continue maximale, et dans lequel le ROD pour le concentrateur d'oxygène n'est pas inférieur à environ 110 litres.

8. Concentrateur d'oxygène (10) selon la revendication 1 dans lequel un ratio RODW du concentrateur d'oxygène (10) est déterminé comme: RODW = (durée de production^{∗}d'O2)/poids total du concentrateur d'oxygène, où la durée représente la durée de vie du concentrateur d'oxygène sur une seule charge d'une ou de plusieurs batteries durant des conditions de fonctionnement de production d'oxygène continue maximale, et dans lequel le RODW pour le concentrateur d'oxygène n'est pas inférieur à environ 0,48 (lpm-hr)/kg (0,22 (lpm-hr)/lbs).

9. Procédé pour la concentration d'oxygène utilisant le concentrateur d'oxygène selon la revendication 1, dans lequel la puissance est fournie au concentrateur d'oxygène uniquement à travers une ou plusieurs batteries (361A) ayant une autonomie en une seule charge supérieure à environ 1,5 heures durant des conditions de fonctionnement de production d'oxygène continue maximale, le procédé comprenant les étapes de:
génération d'une alimentation en air comprimé depuis l'air ambiant via le concentrateur;
génération d'une alimentation en gaz enrichi en oxygène à partir de l'alimentation en gaz comprimé via les lits à tamis;
équilibrage des lits à tamis de sorte que l'un desdits lits à tamis soit à ladite pression intermédiaire via la vanne d'équilibrage en communication fluidique avec les lits à tamis; et
transmission du gaz enrichi en oxygène de l'alimentation générée du gaz enrichi en oxygène à un circuit respiratoire pour transmission à une voie aérienne d'un sujet, dans lequel un ratio ROW est déterminé comme: ROW = (production d'O2) /poids total du concentrateur d'oxygène, dans lequel la production d'O2 est l'oxygène continu maximal transmis au circuit respiratoire, dans lequel le ratio ROW est supérieur à environ 0,42 lpm/kg (0,19 lpm/lbs).

10. Procédé selon la revendication 9, dans lequel le concentrateur d'oxygène a un poids total inférieur à environ 4,54 kg (10 lbs).

11. Procédé selon la revendication 9, comprenant en outre la fourniture d'une alimentation électrique au concentrateur d'oxygène uniquement par le biais d'une ou de plusieurs batteries, dans lequel une autonomie d'une batterie en une seule charge d'une ou de plusieurs batteries lorsqu'une ou plusieurs batteries représentent la seule source d'alimentation électrique pour le concentrateur durant des conditions de fonctionnement de production d'oxygène continue maximale est supérieure à environ 1,5 heures.

12. Procédé selon la revendication 9, dans lequel le concentrateur d'oxygène a un volume total inférieur à environ 10,5 litres (640 pouces cubiques).

13. Procédé selon la revendication 11, dans lequel un ratio de ROD est déterminé comme: ROD = (durée de production^{∗}d'O2), où la durée représente la durée de vie du concentrateur d'oxygène sur une seule charge d'une ou de plusieurs batteries durant des conditions de fonctionnement de production d'oxygène continue maximale, et où le ROD pour le concentrateur d'oxygène n'est pas inférieur à environ 110 litres.

14. Procédé selon la revendication 11, dans lequel un ratio de ROW est déterminé comme: RODW = (durée de production^{∗} d'O2), où la durée représente la durée de vie du concentrateur d'oxygène sur une seule charge d'une ou de plusieurs batteries durant des conditions de fonctionnement de production d'oxygène continue maximale, et dans lequel le RODW pour le concentrateur d'oxygène n'est pas inférieur à environ 0,48 (lpm-hr) /kg. (0,22 (lpm-hr) /lbs).
